# EUROPEAN PATENT APPLICATION

(11) **EP 1 900 371 A1**
(43) Date of publication of application: **19.03.2008**
(21) Application number: 07015970.2
(22) Date of filing: 14.08.2007
(51) Int. Cl.: A61K 36/074

(54) **Method for treating and/or preventing intestinal bacterial imbalance**

(30) Priority: 14.08.2006 US 503175
(71) Applicant: Kindway International Ltd., Causeway Bay Hong Kong (CN)
(72) Inventor: Chung, Chee-Keung, Kwun Town, Kowloon (HK)
(74) Representative: Oser, Andreas

(57) **Abstract**

The present invention provides a method for treating or preventing intestinal bacterial imbalance (IBI) in a mammal. The method includes orally administering a composition comprising germination activated sporoderm-broken *Ganoderma lucidum* spore powder ("GLSs") or a mixture of GLSs and beneficial intestinal bacteria, such as *Lactobacillus bifidus,* to a mammal suffering from IBI or having a tendency to develop IBI.

## Description

### FIELD OF THE INVENTION

This invention relates to a method for treating and/or preventing intestinal bacteria imbalance (IBI) in a mammal. Specifically, the method includes orally administering germination activated sporoderm-broken *Ganoderma lucidum* spores ("CILSs") or a combination of GLSs and beneficial intestinal bacteria to a mammal suffering from IBI or having a tendency to develop IBI.

### BACKGROUND OF THE INVENTION

Humans carry an estimated two to four pounds of bacteria in their intestinal tract. Many of the bacteria found in the digestive tracts are beneficial to humans. For example, some bacteria help to make vitamin K and some other bacteria help our immune system function properly. These beneficial bacteria live on the intestinal walls among thousands of protruding fingers, called villi, where they support digestion and prevent over-colonization with pathogenic (disease-causing) bacteria such as *Escherichia coli* and *Clostridium difficile.* The beneficial and pathogenic bacteria form a constantly changing balance as they compete to take hold and remain in the intestine.

The delicate balance between the beneficial and pathogenic bacteria can be affected by a number of factors such as stress, disease, contaminated foods, and antibiotics. For example, a common cause of intestinal bacteria imbalance (IBI) is antibiotic therapy. Antibiotics kill off beneficial and pathogenic bacteria indiscriminately, allowing the more aggressive pathogenic bacteria-which are usually kept in check by an 85:15 ratio with beneficial bacteria-to grow out of control.

IBI can cause inflammation in the mucosal layer of the intestine, excessive gas, bloating, diarrhea or constipation and poor nutrient absorption. Moreover, it can leave undigested food in the intestine, where it can putrefy (rot) and release toxins that invade the body and compromise health. IBI has been linked with disorders like yeast infections, irritable bowel syndrome and rheumatoid arthritis.

One approach to keeping an healthy intestinal microflora is to dietarily supplement beneficial bacteria to the human. For example, *Lactobacillus,* which is one genus of many friendly intestinal microflora and exists in most diary products, is considered to be especially beneficial to mammals in the ability to aid in the breakdown of proteins, carbohydrates and fats in food, and help absorption of necessary elements and nutrients such as minerals, amino acids and vitamins required for humans and other animals to survive.

Ganoderma belongs to the Polyporaceae group of the Fungi family. Ganoderma is widely used in traditional Chinese medicine as an auxiliary treatment for a variety of medical conditions, such as hepatis, AIDS, cancer, and autoimmune diseases. The germination activated sporoderm-broken Ganoderma lucidum spores ("GLSs") are the essence of Ganoderma and contain active ingredients such as polysaccharides, sterols, oleic acid, linoleic acid, triterpenes, ceramides and certain organic ions, all of which possessing strong bioactivity. The GLSs have also been used for the treatment of neurological disorders. To date, however, there have been no reports on the effects of GLSs on IBI.

### SUMMARY OF THE INVENTION

The present invention provides a method for treating intestinal bacterial imbalance (IBI) in a mammal. The method comprises administering to a mammal which has IBI an effective amount of germination activated sporoderm-broken *Ganoderma lucidum* spores (GLSs). The IBI can be caused by stress, antibiotic treatment, an imbalanced diet or being exposed to contaminated food, or suffering from certain disorders, such as yeast infections, irritable bowel syndrome and rheumatoid arthritis. The most noticeable cause for IBI is due to antibiotic treatment, such as lincomycin hydrochloride treatment.

The effective amount of GLSs to be used for treatment of IBI is between 0.005 and 20 g / kg body weight / day.

Additionally, the GLSs are preferred to be co-administered with an effective amount of beneficial intestinal bacteria. Examples of the beneficial intestinal bacteria include *Lactobacilli* or *Bifidobacteria* or a combination thereof. The preferred beneficial intestinal bacteria are *Lactobacillus bifidus.* The preferred amount of the beneficial intestinal bacteria to be used in conjunction with GLSs is between 0.02 and 20 g / kg body weight / day.

The GLSs alone, or in combination with the beneficial intestinal bacteria, can raise the intestinal aerobic and anaerobic bacterial counts in the mammal.

The present invention also provides a method for preventing an intestinal bacterial imbalance (IBI) in a mammal, which comprises administering to the mammal an effective amount of germination activated sporoderm-broken *Ganoderma lucidum* spores (GLSs). The mammal has a tendency to develop the IBI, such as the mammal is under antibiotic treatment. The GLSs are preferred to be administered to the mammal prior to or concurrently with the antibiotic treatment. Additionally, the GLSs are preferably co-administered with an effective amount of beneficial intestinal bacteria, such as *Lactobacilli* or *Bifidobacteria* or a combination thereof. The preferred beneficial intestinal bacteria are *Lactobacillus bifidus.*

GLSs alone, or in combination with the beneficial intestinal bacteria, can raise the intestinal aerobic and anaerobic bacterial counts in the mammal.

### BRIEF DESCRIPTION OF DRAWINGS

Figure 1 is a microscopic picture showing Haematoxylin & Eosin (HE) staining of a paraffin section of intestinal mucosa from a mouse in the normal control group, amplification 200x
Figure 2 is a composite of microscopic pictures showing HE staining of paraffin sections of intestinal mucosa from mice in the IBI control group, amplification 200x.
Figure 3 is a microscopic picture showing HE staining of a paraffin section of intestinal mucosa from a mouse in the low dose GLSs control group, amplification 200×.
Figure 4 is a microscopic picture of HE staining showing a paraffin section of intestinal mucosa from a mouse in the medium dose GLSs control group, amplification 200×..
Figure 5 is a microscopic picture showing HE staining of a paraffin section of intestinal mucosa from a mouse in the high dose GLSs control group, amplification 200×.
Figure 6 is a composite of microscopic pictures showing HE staining of paraffin sections of intestinal mucosa from mice in the low dose *Lactobacillus bifidus* control (LB control) group, amplification 200×.
Figure 7 is a composite of microscopic pictures showing HE staining of paraffin sections of intestinal mucosa from mice in the medium dose LB control group, amplification 200×.
Figure 8 is a composite of microscopic pictures showing HE staining of paraffin sections of intestinal mucosa from mice in the high dose LB control group, amplification 200x.
Figure 9 is a composite of microscopic pictures showing HE staining of paraffin sections of intestinal mucosa from mice in the low dose *GLSs-Lactobacillus bifidus* mixture control (GLSs-LB control) group, amplification 200×.
Figure 10 is a composite of microscopic pictures showing HE staining of paraffin sections of intestinal mucosa from mice in the medium dose GLSs-LB control group, amplification 200×.
Figure 11 is a composite of microscopic pictures showing HE staining of paraffin sections of intestinal mucosa from mice in the high dose GLSs-LB control group, amplification 200×.
Figure 12 is a composite of microscopic pictures showing HE staining of paraffin sections of intestinal mucosa from mice in the low dose LB treatment group.
Figure 13 is a composite of microscopic pictures showing HE staining of paraffin sections of intestinal mucosa from mice in the medium dose LB treatment group.
Figure 14 is a composite of microscopic pictures showing HE staining of paraffin sections of intestinal mucosa from mice in the high dose LB treatment group, amplification 200x.
Figure 15 is a composite of microscopic pictures showing HE staining of paraffin sections of intestinal mucosa from mice in the low dose GLSs treatment group, amplification 200×.
Figure 16 is a composite of microscopic pictures showing HE staining of paraffin sections of intestinal mucosa from mice in the medium dose GLSs treatment group, amplification 200x.
Figure 17 is a composite of microscopic pictures showing HE staining of paraffin sections of intestinal mucosa from mice in the high dose GLSs treatment group, amplification 200×.
Figure 18 is a composite of microscopic pictures showing HE staining of paraffin sections of intestinal mucosa from mice in the low dose GLSs-LB treatment group, amplification 200×.
Figure 19 is a composite of microscopic pictures showing HE staining of paraffin sections of intestinal mucosa from mice in the medium dose GLSs-LB treatment group, amplification 200×.
Figure 20 is a composite of microscopic pictures showing HE staining of paraffin sections of intestinal mucosa from mice in the high dose GLSs-LB treatment group, amplification 200×.
Figure 21 is a composite of microscopic pictures showing HE staining of paraffin sections of intestinal mucosa from mice in the low dose LB pretreatment group, amplification 200x.
Figure 22 is a composite of microscopic pictures showing HE staining of paraffin sections of intestinal mucosa from mice in the medium dose LB pretreatment group, amplification 200×.
Figure 23 is a composite of microscopic pictures showing HE staining of paraffin sections of intestinal mucosa from mice in the high dose LB pretreatment group, amplification 200x.
Figure 24 is a composite of microscopic pictures showing HE staining of paraffin sections of intestinal mucosa from mice in the low dose GLSs pretreatment group, amplification 200x.
Figure 25 is a composite of microscopic pictures showing HE staining of paraffin sections of intestinal mucosa from mice in the medium dose GLSs pretreatment group, amplification 200×.
Figure 26 is a composite of microscopic pictures showing HE staining of paraffin sections of intestinal mucosa from mice in the high dose GLSs pretreatment group, amplification 200x.
Figure 27 is a composite of microscopic pictures showing HE staining of paraffin sections of intestinal mucosa from mice in the low dose GLSs-LB pretreatment group, amplification 200x.
Figure 28 is a composite of microscopic pictures showing HE staining of paraffin sections of intestinal mucosa from mice in the medium dose GLSs-LB pretreatment group, amplification 200x.
Figure 29 is a composite of microscopic pictures showing HE staining of paraffin sections of intestinal mucosa from mice in the high dose GLSs-LB pretreatment group, amplification 200×.

### DETAILED DESCRIPTION OF THE INVENTION

There are a variety of harmful microorganisms that live and form microorganic flora in an intestinal tract of humans and animals. There are also many beneficial microorganisms that give beneficial effects to the hosts. In a healthy environment, the harmful and the beneficial microorganisms keep symbiotic or antagonistic relationship in the body. However, the outgrowth of the harmful microorganisms breaks up the balance between the harmful and the beneficial microorganisms and thus causes an intestinal bacterial imbalance (IBI).

When the harmful microorganisms (such as bacteria and yeast) become overgrown in the intestinal tract, the host has a condition called dysbiosis. In humans, dysbiosis has been linked with disorders such as yeast infections, irritable bowel syndrome and rheumatoid arthritis.

Yeast are unicellular fungi. Yeast organisms are always present in all people, but are usually prevented from "overgrowth" (uncontrolled multiplication resulting in symptoms) by naturally occurring microorganisms. Yeast infection, also called candidiasis, is commonly caused by *Candida albicans.* Candidiasis is one of the most common causes of vaginal irritation or vaginitis, and can also occur on the male genitals, particularly in uncircumcised men. At least three quarters of all women will experience candidiasis at some point in their lives. The *Candida albicans* organism is found in the vaginas of almost all women and normally causes no problems. However, when it gets out of balance with the other "normal flora," such as lactobacilli, an overgrowth and symptoms can result. Pregnancy, the use of oral contraceptives and some antibiotics, and diabetes mellitus can lead to an increased incidence in yeast infections.

In gastroenterology, irritable bowel syndrome (IBS) or spastic colon is a functional bowel disorder characterized by abdominal pain and changes in bowel habits which are not associated with any abnormalities seen on routine clinical testing. It is fairly common and makes up 20-50% of visits to gastroenterologists. Lower abdominal pain, and bloating associated with alteration of bowel habits and abdominal discomfort relieved with defecation are the most frequent symptoms. The abdominal pain type is usually described in a patient as either diarrhea-predominant (IBS-D), constipation-predominant (IBS-C) or IBS with alternating stool pattern (IBS-A). In some individuals, IBS may have an acute onset and develop after an infectious illness characterized by two or more of the following: fever, vomiting, acute diarrhea, and positive stool culture. This post infective IBS has been termed "Post Infectious IBS" (IBS-PI). This condition is more homogenous, being mostly IBS-D and is drawing much clinical investigation.

IBS is highly prevalent in the Western world, but despite the advancement of many theories, no clear cause has yet been established. Recent studies found that 78% to 84% of patients with IBS had bacterial overgrowth. Pimentel M et al., Am J Gastroenterol, 95(12): 3503-3506 (2000); Pimentel M et al., Am J Gastroenterol. 98(2): 412-419 (2003). Subsequent studies have also identified significant bacterial overgrowth and demonstrated substantial reduction in symptoms following treatments, especially with antibiotics specific to the strains that are in excess.

Another common cause of dysbiosis is associated with antibiotic therapy in treating patients with inflammatory rheumatoid arthritis. Antibiotic therapy was first introduced by Thomas McPherson Brown, M.D. (1906-1989), a well known rheumatologist, who pioneered this treatment based on the findings that inflammatory rheumatic diseases, such as rheumatoid arthritis, scleroderma, lupus, juvenile rheumatoid arthritis, polymyositis, ankylosing spondylitis, etc., have an infectious cause such as mycoplasma and other bacterial L forms. The significance of this therapy is the use of low dose antibiotics, particularly from the tetracycline or macrolide families, to attack the disease process at its source, namely the infectious agent. In contrast to the treatment of ordinary, acute bacterial infections with faster growing bacteria, the bacterial forms which trigger the chronic infectious disease processes are much slower growing organisms; thus, the antibiotic protocols prescribed for treating the rheumatoid diseases are based on the use of long-term, low-dose antibiotics, usually given only three days per week - sometimes more frequently.

The beneficial or "friendly" intestinal bacteria perform many important bodily functions. For example, some of these beneficial intestinal bacteria can ameliorate gastrointestinal conditions such as diarrhea or constipation; some of them can prevent cancer and improve the body's resistance to infection through activation of immune system; and some of them act to suppress the metabolic production produced by the harmful microorganisms, such as ammonia, hydrogen sulfide, amines, or carcinogen. The most widely known beneficial intestinal bacteria include the genera of *Lactobacillus* and *Bifidobacterium.*

*Lactobacillus* is a genus of Gram-positive facultative bacteria. A facultative bacterium is usually an anaerobic bacterium that makes ATP by aerobic respiration if oxygen is present but is also capable of switching to fermentation under anaerobic conditions. Lactobacilli are a major part of the lactic acid bacteria group, named as such because most of its members convert lactose and other simple sugars to lactic acid. They are common and usually benign, even necessary, inhabitants of humans and other animals. In humans, they are present in the vagina and the gastrointestinal tract, and are an important genus of the gut flora. Many species of *Lactobacillus* are prominent in decaying plant material. The production of lactic acid makes its environment acidic which inhibits the growth of some harmful bacteria. The most commonly known Lactobacillus sp. include *L. acidophilus, L. bifidus, L. bulgaricus, L. casei, L. delbrueckii, L. fermentum, L. plantarum, L. reuteri, L. agilis, L. aviarius, L. amylovorus, L. brevis, L. crispatus, L. gallinarum, L. gasseri, L. johnsonii, L. murinus, L. hamsteri, L. intestinalis, L. ruminis,* and *L. salivarius.*

Some *Lactobacillus* species are used industrially for the production of yogurt, sauerkraut, pickles, and other fermented foods, such as silage.' Some yogurt drinks contain *Lactobacillus* bacteria as a dietary supplement. Korean kimchi is also made using lactic acid fermentation techniques. Many lactobacilli are unique among living things as they do not require iron for growth and have an extremely high hydrogen peroxide tolerance.

*Bifidobacterium* is a genus of a Gram-positive, anaerobic, branched rod-shaped bacterium. In the intestines, they ferment sugars to produce lactic acid. *Bifidobacteria,* called probiotics, are a natural part of the bacterial flora in the human body and have a symbiotic bacteria-host relationship with humans. For example, *B. longum* promotes good digestion, boosts the immune system, and produces lactic and acetic acid that controls intestinal pH. These bacteria also inhibit the growth of *Candida albicans, E. coli,* and other bacteria that have more pathogenic qualities than *Bifidobacteria.* It is thought that *Bifidobacterium's* ability to compete with other gastrointestinal bacteria and occupy a large percentage in the bacterial flora of the gastrointestinal region might be partly due to the large variety of molecules that it is able to use for energy. Schell, Mark A., et al., Proc Natl Acad Sci USA, 99(22): 14422-14427 (2002). The most commonly known Bidifobacteria include *B. angulatum; B. animalis; B. asteroides; B. bifidum; B. boum; B. breve; B. catenulatum; B. choerinum; B. coryneforme; B. cuniculi; B. dentium; B. gallicum; B. gallinarum; B indicum; B. longum; B. magnum; B. merycicum; B. minimum; B. pseudocatenulatum; B. pseudolongum; B. psychraerophilum; B. pullorum; B. ruminantium; B. saeculare; B. scardovii; B. simiae; B. subtile; B. thermacidophilum; B. thermophilum; B. urinalis.*

The term "probiotics" is now generally accepted to mean "a microorganism and/or a substance that give beneficial effect to a host through control of intestinal flora." Parker, R B: An. Nutr. Health, 29, 4-8 (1974). Sometime, probiotics can be referred to beneficial bacteria that can be found in various foods which can add to the intestinal tract to improve the intestinal bacterial balance. In any case, a substance that is called probiotics has a function of relieving intestinal disorders.

Currently, there are three probiotics that are commercially available, they are *Lactobacillus acidophilus, Lactobacillus bifidus,* and *Streptococcus faecium.*

Various cultures of acidophilus are available in many stores, particularly health food stores, as powders, capsules, tablets, and liquids and measured by the amount of viable bacteria per dosage. There are many claims for the use of acidophilus, though it is best known for reimplanting friendly bacteria into the colon to assure return of bodily functions after a course of antibiotic drugs. In fact, acidophilus itself acts as a mild antibiotic-that is, it has antibacterial activity. With regular use, it may even replace harmful bacteria in the colon or vaginal tract of women, where acidophilus is also commonly used to treat yeast infections. It is further employed as part of the treatment for intestinal yeast overgrowth and the many symptoms that this may generate. These bacteria also help in the production of some B vitamins and vitamin K and in the breakdown of various foods.

*Lactobacillus bifidus* has become part of intestinal bioculture treatment, often along with acidophilus. The bifidus culture is more prevalent in infants, often as their first organism, but can also be an important part of the adult gastrointestinal tract. Like acidophilus, it helps in the synthesis of B vitamins, in food digestion, and in inhibiting the growth of the coliform bacteria and possibly more pathogenic colon bacteria, such as salmonellae.

*Streptococcus faecium* is another important colon bacterium that has received recent attention. Its actions are similar to those of acidophilus. It is important in B vitamin biosynthesis, aids the digestion of foods, likely by producing certain enzymes, and inhibits other, more toxic bacteria; thus supplementation with S. *faecium* may help in some cases of diarrhea. S. *faecalis,* a potentially pathogenic bacterium, has been listed by mistake instead of S. faecium on some bacterial replacement products.

These three bacteria may be taken individually and alternated weekly or every couple of weeks. They can also be taken all together (there are some products that contain all three) on a regular basis when used to balance the effect of one or more courses of antibiotics. There is a possibility that the combination of bacteria works better to rebalance colon health than the individual organisms.

The present invention provides a method for preventing or ameliorating intestinal bacterial imbalance (IBI) in a mammal by administering germination activated sporoderm-broken *Ganoderma lucidum* spores (GLSs) to the mammal. In this study, an animal model was established to mimic the IBI in humans due to antibiotic therapy. This model is used to evaluate the impact of oral administration of GLSs, *Lactobacillus bifidus,* or a combination of GLSs and *Lactobacillus bifidus* on intestinal microflora under normal and diseased conditions. The animal model involves giving normal mice gastric perfusion of lincomycin hydrochloride for six consecutive days to induce IBI. This model is well-recognized in the enterogastric field to produce symptoms similar to those of IBI in humans.

Lincomycin is a systemic antibiotic that comes from the bacteria *Streptomyces lincolnensis.* It has been structurally modified to its more commonly known 7-chloro-7-deoxy derivative, clindamycin. Although similar in structure, antibacterial spectrum, and in mechanism of action to macrolides they are also effective against other species as well, *i.e.,* actinomycetes, mycoplasma, and some species of *plasmodium.* However, because of its adverse effect and toxicity, it is rarely used today and reserved for patients who are either allergic to penicillin or where bacteria has developed resistance.

Animal models have been widely used to study host-bacterial interactions for intestinal microflora. For example, animal models of irritable bowel disease (IBD) have provided the strongest evidence for the role of resident luminal bacteria in disease pathogenesis and have been used to test the therapeutic efficacy of probiotics (single or in combination, and in different doses). Based on the animal model data, several clinical trails of probiotics have been initiated in patients with IBD. Mahida YR et al., Clinical Science, 107:331-341 (2004).

As will be shown in the following examples, *infra,* IBI-related symptoms and pathological changes in intestinal tissue can be prevented or ameliorated by oral administration of GLSs or a combination of GLSs and *Lactobacillus bifidus.*

In describing preferred embodiments of the present invention, specific terminology is employed for the sake of clarity. However, the invention is not intended to be limited to the specific terminology so selected. It is to be understood that each specific element includes all technical equivalents which operate in a similar manner to accomplish a similar purpose.

One aspect of the present invention relates to a method for preventing or treating IBI in a mammal. The method comprises administering to a mammal an effective amount of GLSs.

GLSs is a brown powder that is slightly soluble in water. Bioactive GLSs can be produced in a process containing the following steps:
*I. Induction of germination:* Mature and perfect spores of *Ganoderma lucidum* are carefully selected to undergo a soaking process to induce germination. Spores are kept in clear or distilled water, biological saline solution, or other nutritional solutions that could enable the spores of *Ganoderma lucidum* to germinate rapidly. Examples of nutritional solutions include coconut juice or a 1-5% malt extract solution, 0.5-25% extracts of *Ganoderma lucidum* sporocarps or *Ganoderma lucidum* capillitia, a solution containing 0.1-5% biotin, and a solution containing 0.1-3% potassium phosphate (monobasic) and magnesium sulfate. The choice of solution would depend on the soaking time required, the amount of spores to be processed and other such factors as availability of materials. One or more of the above germination solutions could be used, with the amount added being 0.1 -5 times the weight of the spores of *Ganoderma lucidum.* The soaking time can be determined according to the temperature of the water, and usually the soaking was carried out for 30 min to 8 hours with the temperature of the water at 20-43°C. Preferably, the soaking time is 2-4 hours, and the temperature of water is 25-35°C.
*II. Activation culture:* The spores of *Ganoderma lucidum* are removed from the soaking solution and excess solution is eliminated by allowing it to drip. The spores are then placed in a well-ventilated culturing box at a constant temperature and humidity so that spore culture activation could be carried out. The relative humidity of the culture is generally set at 65-98%, the culture temperature set at 18-48°C and the activation time may last from 30 min to 24 hours. Preferably humidity is 85-97% and temperature is 25-35°C. During activation, the cell walls of the spores of *Ganoderma lucidum* are clearly softened such that it is easier to penetrate the cell walls of the spores. The activation of spores of *Ganoderma lucidum* typically reaches a rate of more than 95%.
*III. Treatment of the epispores:* After the germination/activation process, the spores are treated by enzymolysis. This process is carried out at a low temperature and under conditions such that enzyme activity is maintained, using chitinase, cellulase, or other enzymes, which are commonly used in the industry. The process is complete when the epispores lost their resilience and became brittle. Alternatively, physical treatments are carried out to penetrate the cell walls, for example, micronization, roll pressing, grinding, super high pressure microstream treatment, and other mechanical methods commonly used in the industry could be carried out, with a penetration rate of over 99%.
*IV. Drying or extraction:* Drying is carried out at low temperature using standard methods including freeze-drying or vacuum-drying etc., which are commonly used in the industry. The obtained product has a moisture content less than 4%. After drying, the bioactive substances are extracted by water or alcohol, or by thin film condensation. The extracted bioactive substances can be further purified by dialysis to ensure no contamination in the final products. The final product can be made into purified powders, extract pastes, solutions for injection, or for oral consumption.

A more detailed description for production of GLSs can be found in U.S. Patent No. 6,316,002, which is hereby incorporated by reference. GLSs are also commercially available from Kindway International Limited/Holistol International Limited in Hong Kong.

The effective amount of GLSs is a dosage which is useful for preventing or ameliorating IBI-related symptoms and pathogenesis. Toxicity and therapeutic efficacy of GLSs can be determined by standard pharmaceutical procedures in cell culture or experimental animal models, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. The data obtained from cell culture assays and animal studies can be used in formulating a range of dosages for use in humans.

Generally, appropriate dosages for administering GLSs may range, for example, from about 0.005 g / kg body weight / day to about 20 g / kg body weight / day. In one embodiment, the effective amount of GLSs is between 0.05 and 10 g / kg body weight /day. In another embodiment, the effective amount of GLSs is between 0.5 and 5 g / kg body weight / day.

GLSs are preferred to be administered orally. The administration can be in one dose, or at intervals such as three times daily, twice daily, once daily, once every other day, or once weekly. A typical treatment regimen is one administration per day for a period of two or more days, preferably 3-14 days. Dosage schedules for administration of GLSs can be adjusted based on the individual conditions and needs of the target. Continuous infusions may also be used after the bolus dose. The effects of any particular dosage can be monitored by suitable bioassays.

GLSs may be administered with one or more beneficial bacteria. Examples of beneficial bacteria include, but are not limited to, the genus of *Lactobacillus,* such as *L. acidophilus, L. bifidus, L. bulgaricus, L. casei, L. delbrueckii, L. fermentum, L. plantarum, L. reuteri, L. agilis, L. aviarius, L. amylovorus, L. brevis, L. crispatus, L. gallinarum, L. gasseri, L. johnsonii, L. murinus, L. hamsteri, L. intestinalis, L. ruminis,* and *L. salivarius;* the genus of Bifidobacterium, such as *B. angulatum; B. animalis; B. asteroides; B. bifidum; B. boum; B. breve; B. catenulatum; B. choerinum; B. coryneforme; B. cuniculi; B. dentium; B. gallicum; B. gallinarum; B indicum; B. longum; B. magnum; B. merycicum; B. minimum; B. pseudocatenulatum; B. pseudolongum; B. psychraerophilum; B. pullorum; B. ruminantium; B. saeculare; B. scardovii; B. simiae; B. subtile; B. thermacidophilum; B. thermophilum; B. urinalis.* Additionally, *Streptococcus faecium* can be added along or in any combination of *Lactobacilli* or *Bifidobacteria* sp.

The effective amount of beneficial bacteria is a dosage which is useful for preventing or ameliorating IBI-related symptoms and pathogenesis. Appropriate dosages for beneficial bacteria may range, for example, from about 0.02 g / kg body weight / day to about 20 g / kg body weight / day. In one embodiment, the effective amount of beneficial bacteria is between 0.2 and 20 g / kg body weight / day. In another embodiment, the effective amount of beneficial bacteria is between 1 and 10 g /kg body weight / day.

GLSs can also be formulated into a pharmaceutical composition with a pharmaceutically acceptable carrier. As used herein the language "pharmaceutically acceptable carrier" is intended to include any and all solvents, solubilizers, fillers, stabilizers, binders, absorbents, bases, buffering agents, controlled release vehicles, diluents, emulsifying agents, humectants, lubricants, dispersion media, coatings, antibacterial or antifungal agents, isotonic and absorption delaying agents, and the like, compatible with pharmaceutical administration. The use of such media and agents for pharmaceutically active substances is well known in the art. Except insofar as any conventional media or agent is incompatible with the active compound, use thereof in the compositions is contemplated. Supplementary agents can also be incorporated into the compositions.

The pharmaceutical composition of the present invention is formulated to be compatible with its intended route of administration, e.g., oral or parenteral administration. Oral compositions generally include an inert diluent or an edible carrier. They can be enclosed in gelatin capsules or compressed into tablets. For the purpose of oral therapeutic administration, the active compound can be incorporated with a solid carrier and used in the form of tablets, troches, or capsules. Oral compositions can also be prepared using a fluid carrier. Pharmaceutically compatible binding agents, and/or adjuvant materials can be included as part of the composition. The tablets, pills, capsules, troches and the like can contain any of the following ingredients, or compounds of a similar nature: a binder such as microcrystalline cellulose, gum tragacanth or gelatin; an excipient such as starch or lactose; a disintegrating agent such as alginic acid, Primogel, or corn starch; a lubricant such as magnesium stearate or Stertes; a glidant such as colloidal silicon dioxide; a sweetening agent such as sucrose or saccharin; or a flavoring agent such as peppermint, methyl salicylate, or orange flavoring.

Pharmaceutical compositions of GLSs that are suitable for injectable use include sterile aqueous solutions (where water soluble) or dispersions and sterile powders for the extemporaneous preparation of sterile injectable solutions or dispersion. For intravenous administration, suitable carriers include physiological saline, bacteriostatic water, Cremophor EL™ (BASF, Parsippany, NJ) or phosphate buffered saline (PBS). In all cases, the injectable composition should be sterile and should be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), and suitable mixtures thereof. The proper fluidity can be maintained, for example, by the use of a coating such as lecithin, by the maintenance of the requited particle size in the case of dispersion and by the use of surfactants. Prevention of the action of microorganisms can be achieved by various antibacterial and antifungal agents, for example, parabens, chlorobutanol, phenol, ascorbic acid, thimerosal, and the like. In many cases, it will be preferable to include isotonic agents, such as sodium chloride, sugars, polyalcohols (e.g., manitol, sorbitol, *etc.*) in the composition. Prolonged absorption of the injectable compositions can be brought about by including in the composition an agent which delays absorption, for example, aluminum monostearate and gelatin.

Sterile injectable solutions can be prepared by incorporating the GLSs in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying which yields a powder of the active ingredient plus any additional desired ingredient from a previously sterile-filtered solution thereof.

It is especially advantageous to formulate oral or parenteral compositions in dosage unit form for ease of administration and uniformity of dosage. Dosage unit form as used herein includes physically discrete units suited as unitary dosages for the subject to be treated; each unit contains a predetermined quantity of active compound calculated to produce the desired therapeutic effect in association with the required pharmaceutical carrier. The specification for the dosage unit forms of the invention are dictated by and directly dependent on the unique characteristics of the active compound and the particular therapeutic effect to be achieved, and the limitations inherent in the art of compounding such an active compound for the treatment of individuals. The pharmaceutical compositions can be included in a container, pack, or dispenser together with instructions for administration.

A person or animal suffering from IBI can be diagnosed by standard clinical procedures such as stool analysis. It is also well-recognized that people under antibiotic treatment, under constant stress, having an imbalanced diet or being exposed to contaminated food, or suffering from certain disorders, such as yeast infections, irritable bowel syndrome and rheumatoid arthritis, have a tendency to develop IBI.

The following experimental designs and result are illustrative, but not limiting the scope of the present invention. Reasonable variations, such as those occur to reasonable artisan, can be made herein without departing from the scope of the present invention. Also, in describing the invention, specific terminology is employed for the sake of clarity. However, the invention is not intended to be limited to the specific terminology so selected. It is to be understood that each specific element includes all technical equivalents which operate in a similar manner to accomplish a similar purpose.

### EXPERIMENTAL DESIGN

### I. Materials and methods

*Ganoderma spores:* GLSs were Supplied by the Kindway International Limited/Holistol International Limited, in the form of a capsule containing 0.3g GLSs powder / capsule.

*Experimental animals*: Pure Kunming line mice weighing 20 ± 2 g/each, half of them male and half of them female, were supplied by the Guizhou Province Experimental Animal Center, certificate number: SCXK (Guizhou) 2002-0001.

*MRS culture medium:* MRS culture medium was prepared by mixing the ingredients listed below:

| | | |
|---|---|---|
| Polyvalent peptone 10g | Tryptone 3g | Soluble starch 0.5g |
| Glucose 10g | FeSO₄O 0.1g | MnSO₄ 0.00672g |
| Tween-80 1mL | Cysteine 0.4g | MgSO₄·7H₂O 0.2g |
| KH₂PO₄ 1g | K₂HPO₄ 1g | NaCl 0.01 g |
| Sodium citrate 1g | Yeast extract 5g | Lactose 5g |
| Agarose 15g | Distilled water added to 1000mL | |

The pH value of the culture medium was adjusted to 7.2-7.4 with NaOH. The medium was sterilized at 121°C for 25min.

*Lincomycin hydrochloride:* Lincomycin hydrochloride solution (0.6g/2mL), produced by the Fujian Province Fuqing Pharmaceutical Industrial Company Ltd., was diluted to a final concentration of 100mg/mL with saline.

*Lactobacillus bifidus* culture: *Lactobacillus bifidus* viability was determined by plating 0.1 mL of saline-diluted *Lactobacillus bifidus* culture on MRS solid culture medium, and incubating at 37°C for 24 h. Viable *Lactobacillus bifidus* produce a large number of colonies.

*Dosage:* The clinical recommended dose for GLSs was 1.8g/person/day, which equals a dose of 0.03g/kg/day (assuming an average adult body weight of 60 kg). The maximum dose used in the mouse experiment was 12g/kg/day (high dose group), an amount 400 times over the clinical dose. No death or toxicity was observed during the experimental period. Two other doses: 6.0g/kg (medium dose) and 3.0g/kg (low dose) were also tested.

*Preparation of Ganoderma spore suspension:* well-grounded GLSs were suspended in distilled water to a final concentration of 10%, 5%, and 2.5% (w/v). For example, a 10% suspension was prepared by grinding 10 capsules (3.0g) of GLSs and adding distilled water to the grounded GLSs to a final volume of 30mL. The 10% suspension was then diluted with distilled water to obtain the 5% and 2.5% suspension.

*Preparation of Lactobacillus bifidus suspension: Lactobacillus bifidus* was diluted into 35%; 17.5% and 8.75% (w/v) concentrations with sterile saline.

*Preparation of Ganoderma spores and Lactobacillus bifidus mixtures:* GLSs and *Lactobacillus bifidus* mixtures were prepared by mixing equal volume of 70% *Lactobacillus bifidus* and 20% GLSs. The high concentration mixture (35% *Lactobacillus bifidus* and 10% GLSs) was then diluted to obtain medium 17.5% *Lactobacillus bifidus* and 5% GLSs and low concentration (8.75% *Lactobacillus bifidus* and 2.5% GLSs) mixtures.

*Animal grouping:* Mice were randomly divided into 11 control groups (10 mice per group) and 18 treatment/pretreatment groups (20 mice per group).

The control groups were: the normal control group; the lincomycin hydrochloride control (IBI control) group; three GLSs control groups (high (10%), medium (5%) and low (2.5%) dose groups); three *Lactobacillus bifidus* control (LB control) groups (high (35%), medium (17.5%) and low (8.75%) dose groups); and three *GLSs-Lactobacillus bifidus* mixture control (GLSs-LB control) groups (high (10% + 35%), medium (5% + 17.5%), and low (2.5% + 8.75%) dose groups).

The treatment groups were: three GLSs treatment groups (high, medium, and low dose groups); three *Lactobacillus bifidus* treatment (LB treatment) groups (high, medium, and low dose groups); three GLSs - *Lactobacillus bifidus* mixture treatment (GLSs-LB treatment) groups (high, medium, and low dose groups).

The pretreatment (or prevention) groups were: three GLSs pretreatment groups (high, medium, and low dose groups); three *Lactobacillus bifidus* pretreatment (LB pretreatment) groups (high, medium, and low dose groups); and three GLSs - *Lactobacillus bifidus* mixture pretreatment (GLSs-LB pretreatment) groups (high, medium, and low dose groups).

### Treatment

Normal control group: gastric perfusion of sterile saline at 0.7mL/day for 12 consecutive days.

IBI control group: gastric perfusion of 10mg/mL lincomycin hydrochloride at 0.7 mL/day for 6 consecutive days, followed with distilled water at 0.7mL/day for 6 consecutive days.

GLSs control groups: gastric perfusion of high (10%), medium (5%) and low doses (2.5%) GLSs at 0.7mL/day for 6 consecutive days, followed with distilled water at 0.7mL/day for 6 consecutive days.

LB control groups: gastric perfusion of high (35%), medium (17.5%) and low dose (8.75%) *Lactobacillus bifidus* at 0.7mL/day for 6 consecutive days; followed with distilled water at 0.7mL/day for 6 consecutive days.

GLSs-LB control groups: gastric perfusion of high, medium and low dose GLSs *-Lactobacillus bifidus* mixture at 0.7mL/day for 6 consecutive days, followed with distilled water at 0.7mL/day for 6 consecutive days.

GLSs treatment groups: gastric perfusion of 10mg/mL injectable lincomycin hydrochloride at 0.7mL/day for 6 consecutive days; followed with high (10%), medium (5%), and low dose (2.5%) of GLSs at 0.7mL/day for 6 consecutive days.

LB treatment groups: gastric perfusion of 10mg/mL injectable lincomycin hydrochloride at 0.7mL/day for 6 consecutive days; followed with high (35%), medium (19.5%) and low (8.75%) dose *Lactobacillus bifidus* at 0.7mL/day for 6 consecutive days.

GLSs-LB treatment groups: gastric perfusion of 10mg/mL injectable lincomycin hydrochloride at 0.7mL/day for 6 consecutive days, followed with high, medium and low dose GLSs *-Lactobacillus bifidus* mixture at 0.7mL/day for 6 consecutive days.

GLSs pretreatment groups: gastric perfusion of high (10%), medium (5%) and low dose (2.5%) GLSs at 0.7mL/day for 6 days; followed with 10mg/mL lincomycin hydrochloride at 0.7mL/day for 6 consecutive days.

LB pretreatment groups: gastric perfusion of high (35%), medium (17.5%) and low dose (8.75%) *Lactobacillus bifidus* at 0.7mL/day for 6 consecutive days; followed with 10mg/mL injectable lincomycin hydrochloride at 0.7 mL/day for 6 consecutive days. GS-LB pretreatment groups: gastric perfusion of high, medium and low dose GLSs - *Lactobacillus bifidus* mixture at 0.7mL/day for 6 consecutive days; followed with 10mg/mL injectable lincomycin hydrochloride at 0.7 mL/day for 6 consecutive days.

*General symptoms and body weight observations:* General symptoms of each group of experimental mice were observed each day. Prior to gastric perfusion and upon conclusion of the experiment, body weights were obtained from each mouse in each group.

*Stool specimen collection and bacterial colony counts:* After the experiment was completed for each group of mice, the mice were sacrificed by cervical dislocation. The stool was extracted aseptically from the intestine, placed in a labeled aseptic test tube, weighed, diluted 10-fold with a diluent, completely homogenized and then diluted at 10⁻⁵, 10⁻⁶ and 10⁻⁷ dilutions. 0.1mL of each dilution was plated on culture media and incubated at 37°C for 24 h. The bacterial colonies on each plate were counted and the bacterial count for each specimen was determined (CFU/g specimen). All results were obtained using common logarithmic values and the data were analyzed using statistical methodology.

*Intestinal specimen collection and pathology exams:* After removing stools, the intestine samples were washed in sterile saline, fixed in formalin, embedded in paraffin, sectioned, stained with HE, and observed under a light microscope for pathological changes. Pathological exams and reading of the slides were performed by Assistant Professor Long Yiguo and Assistant Chief Technician Tang Hui from the Department of Pathology at Guiyang Medical College.

### II. Experimental results

*General symptoms:* Mice in the IBI control group showed loose stools, reduced body weight, reduced fur sheen, and other symptoms. After treatment with GLSs, *Lactobacillus bifidus* or both, the stools contained less water, the body weight increased, fur sheen gradually returned, and the mice were more active. Some of the mice in the LB treatment group had loose stools, crested hair and other symptoms, while those in the GS treatment/pretreatment groups, the GS-LB treatment/pretreatment groups, the GS control groups, and the normal control group showed no abnormalities.

*Changes in body weight:* Tables 1 and 2 show the effect of the various treatments on body weight of normal mice.

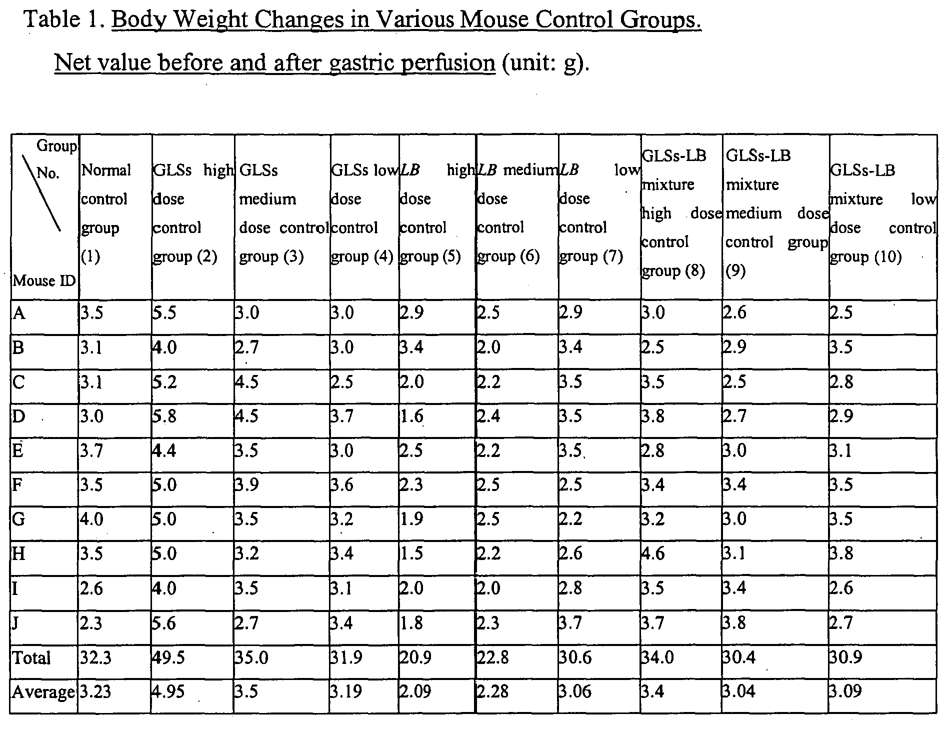

**Table 2. Comparison of Body Weight in the Various Control Groups (t-test).**

| Groups compared | | | Groups compared | |
|---|---|---|---|---|
| 1 vs. 2 | ## | | 3 vs. 10 | |
| 1 vs. 3 | # | | 4 vs. 5 | ## |
| 1 vs. 4 | | | 4 vs. 6 | ## |
| 1 vs. 5 | ## | | 4 vs. 7 | |
| 1 vs. 6 | # | | 4 vs. 8 | |
| 1 vs. 7 | # | | 4 vs. 9 | |
| 1 vs. 8 | | | 4 vs. 10 | |
| 1 vs. 9 | | | 5 vs. 6 | |
| 1 vs. 10 | | | 5 vs. 7 | # |
| 2 vs. 3 | # | | 5 vs. 8 | ## |
| 2 vs. 4 | ## | | 5 vs. 9 | # |
| 2 vs. 5 | ## | | 5 vs. 10 | # |
| 2 vs. 6 | ## | | 6 vs. 7 | # |
| 2 vs. 7 | ## | | 6 vs. 8 | ## |
| 2 vs. 8 | ## | | 6 vs. 9 | # |
| 2 vs. 9 | ## | | 6 vs. 10 | # |
| 2 vs. 10 | ## | | 7 vs. 8 | # |
| 3 vs. 4 | | | 7 vs. 9 | |
| 3 vs. 5 | ## | | 7 vs. 10 | |
| 3 vs. 6 | ## | | 8 vs. 9 | # |
| 3 vs. 7 | # | | 8 vs. 10 | # |
| 3 vs. 8 | | | 9 vs. 10 | |
| 3 vs. 9 | | | | |
| | | | | |

| | | | | |
|---|---|---|---|---|
| Notes: No #: p > 0.05, #: p < 0.05, ##: p < 0.01. | | | | |

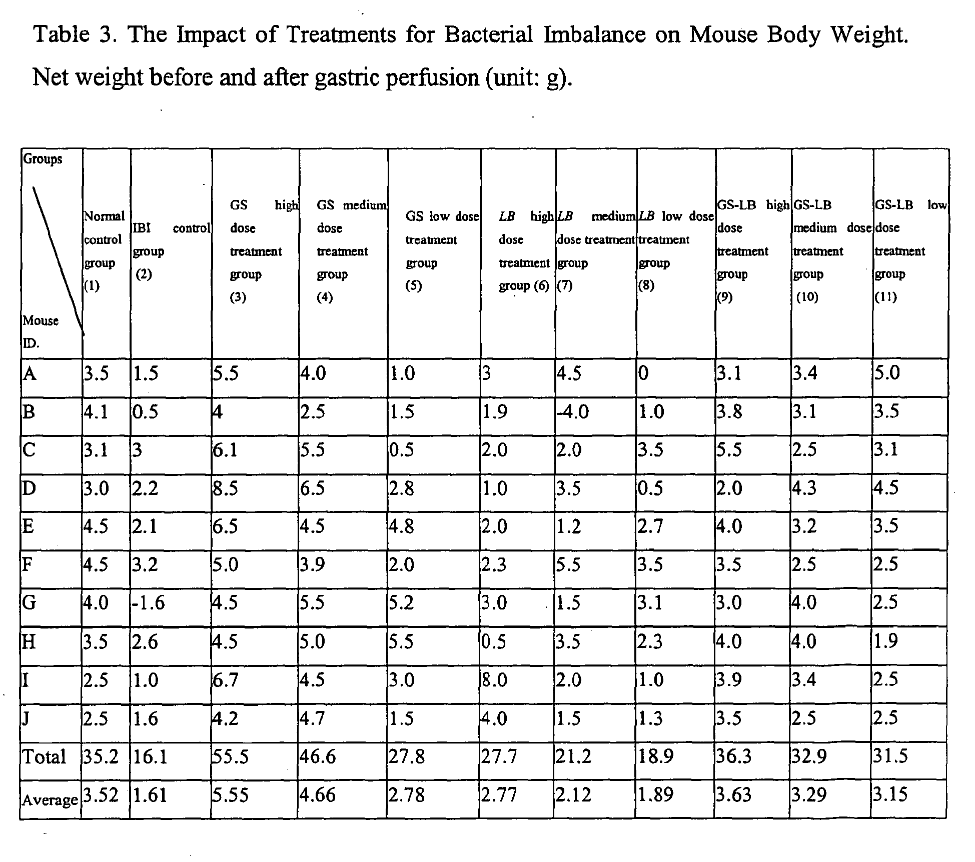

**Table 4. Comparison of Body Weight in Various Treatment Groups (t-test).**

| Groups compared | | | Groups compared | | | Groups compared | | | | |
|---|---|---|---|---|---|---|---|---|---|---|
| 1 vs. 2 | ## | | 2 vs. 7 | # | | 4 vs. 7 | ## | | 6 vs. 10 | ## |
| 1 vs. 3 | ## | | 2 vs. 8 | ## | | 4 vs. 8 | ## | | 6 vs. 11 | ## |
| 1 vs. 4 | # | | 2 vs. 9 | ## | | 4 vs. 9 | # | | 7 vs. 8 | # |
| 1 vs. 5 | ## | | 2 vs. 10 | ## | | 4 vs. 10 | # | | 7 vs. 9 | ## |
| 1 vs. 6 | ## | | 3 vs. 4 | # | | 4 vs. 11 | # | | 7 vs. 10 | ## |
| 1 vs. 7 | ## | | 3 vs. 5 | ## | | 5 vs. 6 | | | 7 vs. 11 | ## |
| 1 vs. 8 | ## | | 3 vs. 6 | ## | | 5 vs. 7 | # | | 8 vs. 9 | ## |
| 1 vs. 9 | | | 3 vs. 7 | ## | | 5 vs. 8 | ## | | 8 vs. 10 | ## |
| 1 vs. 10 | | | 3 vs. 8 | ## | | 5 vs. 9 | ## | | 8 vs. 11 | ## |
| 1 vs. 11 | # | | 3 vs. 9 | ## | | 5 vs. 10 | ## | | 9 vs. 10 | |
| 2 vs. 3 | ## | | 3 vs. 10 | ## | | 5 vs. 11 | ## | | 9 vs. 11 | # |
| 2 vs. 4 | ## | | 3 vs. 11 | ## | | 6 vs. 7 | # | | 10 vs. 11 | |
| 2 vs. 5 | ## | | 4 vs. 5 | ## | | 6 vs. 8 | ## | | | |
| 2 vs. 6 | ## | | 4 vs. 6 | ## | | 6 vs. 9 | ## | | | |

| | | | | | | | | | | |
|---|---|---|---|---|---|---|---|---|---|---|
| Notes: No #: p > 0.05, #: p < 0.05,##: p < 0.01. | | | | | | | | | | |

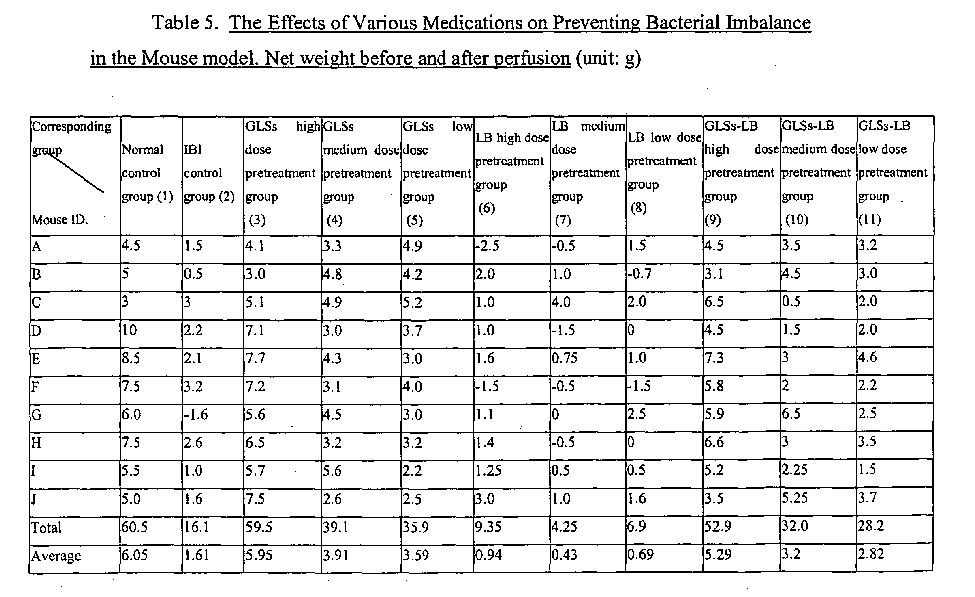

**Table 6. Comparison of Body Weight in the Various Pretreatment Groups (t-test).**

| Groups compared | | | Groups compared | | | Groups compared | | | Groups compared | |
|---|---|---|---|---|---|---|---|---|---|---|
| 1 vs. 2 | ## | | 2 vs. 7 | ## | | 4 vs. 5 | | | 6 vs. 9 | |
| 1 vs. 3 | | | 2 vs. 8 | # | | 4 vs. 6 | ## | | 6 vs. 10 | |
| 1 vs. 4 | # | | 2 vs. 9 | ## | | 4 vs. 7 | ## | | 6 vs. 11 | ## |
| 1 vs. 5 | # | | 2 vs. 10 | ## | | 4 vs. 8 | ## | | 7 vs. 8 | |
| 1 vs. 6 | ## | | 2 vs. 11 | # | | 4 vs. 9 | ## | | 7 vs. 9 | ## |
| 1 vs. 7 | ## | | 3 vs. 4 | # | | 4 vs. 10 | | | 7 vs. 10 | ## |
| 1 vs. 8 | ## | | 3 vs. 5 | # | | 4 vs. 11 | # | | 7 vs. 11 | ## |
| 1 vs. 9 | # | | 3 vs. 6 | ## | | 5 vs. 6 | ## | | 8 vs. 9 | ## |
| 1 vs. 10 | ## | | 3 vs. 7 | ## | | 5 vs. 7 | ## | | 8 vs. 10 | ## |
| 1 vs. 11 | ## | | 3 vs. 8 | ## | | 5 vs. 8 | ## | | 8 vs. 11 | ## |
| 2 vs. 3 | ## | | 3 vs. 9 | | | 5 vs. 9 | ## | | 9 vs. 10 | # |
| 2 vs. 4 | ## | | 3 vs. 10 | # | | 5 vs. 10 | # | | 9 vs. 11 | ## |
| 2 vs. 5 | # | | 3 vs. 11 | ## | | 5 vs. 11 | # | | 10 vs. 11 | |
| 2 vs. 6 | | | | | | | | | | |

| | | | | | | | | | | |
|---|---|---|---|---|---|---|---|---|---|---|
| Notes: No #: p>0.05, #: p<0.05, ##: p<0.01. | | | | | | | | | | |

*Changes in anaerobic intestinal bacteria:* Tables 7 and 8 show the effects of the various treatments on intestinal anaerobic bacteria in normal mice.

**Table 7. The Effect of Various Treatments on Intestinal Anaerobic Bacteria in Normal Mouse (unit: logCFU/g)**

| Sample # | Normal control group (a) | IBI control group (b) | GLSs high dose control group (c) | GLSs medium dose control group (d) | GLSs low dose control group (e) | *LB* high dose control group (f) | *LB* medium dose control group (g) | *LB* low dose control group (h) | GLSs-LB high dose control group (i) | GLSs-LB medium dose control group (j) | GLSs-LB low dose control group (k) |
|---|---|---|---|---|---|---|---|---|---|---|---|
| 1 | 11.28 | 8.55 | 12.69 | 12.18 | 11.31 | 13.71 | 11.3 | 11.7 | 13.66 | 12.63 | 11.62 |
| 2 | 11.07 | 8.63 | 12.59 | 12.21 | 11.53 | 12.94 | 12.39 | 11.66 | 13.57 | 12.59 | 11.82 |
| 3 | 11.47 | 8.25 | 12.87 | 12.11 | 11.23 | 12.95 | 12.48 | 11.64 | 13.87 | 12.45 | 11.68 |
| 4 | 11.24 | 8.54 | 12.82 | 12.86 | 11.43 | 12.23 | 11.47 | 11.36 | 13.82 | 12.67 | 11.74 |
| 5 | 11.77 | 8.43 | 12.82 | 12.15 | 11.23 | 13.13 | 11.37 | 10.84 | 13.37 | 12.73 | 11.79 |
| 6 | 11.56 | 7.96 | 12.87 | 12.63 | 10.96 | 12.65 | 11.56 | 13.11 | 13.11 | 13.15 | 12.23 |
| 7 | 12.96 | 8.25 | 12.69 | 12.53 | 11.85 | 12.85 | 11.47 | 12.36 | 13.45 | 13.25 | 12.42 |
| 8 | 12.12 | 8.12 | 13.14 | 12.54 | 11.56 | 12.42 | 12.04 | 12.54 | 14.21 | 14.15 | 11.39 |
| 9 | 13.1 | 8.32 | 13.23 | 12.41 | 11.54 | 12.30 | 12.31 | 12.74 | 13.56 | 13.68 | 11.48 |
| 10 | 12.2 | 8.54 | 11.58 | 12.63 | 10.84 | 12.41 | 11.48 | 11.96 | 13.42 | 13.46 | 12.45 |
| Total | 116.77 | 83.59 | 127.79 | 124.25 | 113.48 | 127.59 | 117.87 | 119.91 | 136.04 | 130.76 | 117.66 |
| Average | 11.68 | 8.36 | 12.78 | 12. 43 | 11.35 | 12.76 | 11.79 | 11.20 | 13.60 | 13.08 | 11.77 |

**Table 8. Comparison of the Effects of Various treatments on Intestinal Anaerobic Bacteria in Normal Mouse (t-test)**

| Groups compared | | Groups compared | | Groups compared | |
|---|---|---|---|---|---|
| aandb | ## | candd | | e and j | ## |
| a and c | # | c and e | # | e and k | |
| aandd | # | c and f | | f and g | |
| a and e | | c and g | | f and h | # |
| a and f | # | c and h | | f and i | # |
| a and g | | c and i | | f and j | # |
| a and h | | c and j | | f and k | |
| a and i | ## | c and k | | g and h | |
| a and j | ## | d and e | # | g and i | # |
| a and k | | d and f | | g and j | # |
| b and c | ## | d and g | # | g and k | |
| b and d | ## | d and h | # | h and i | ## |
| b and e | ## | d and i | ## | h and j | ## |
| b and f | ## | d and j | ## | h and k | |
| b and g | ## | d and k | | iandj | |
| b and h | ## | e and f | # | i and k | ## |
| b and i | ## | e and g | | j and k | ## |
| b and j | ## | e and h | . | | |
| b and k | ## | e and i | ## | | |

| | | | | | |
|---|---|---|---|---|---|
| Note: # is p < 0.05, ## is p < 0.01. | | | | | |

Table 9 and 10 show the effects of various treatments on intestinal anaerobic bacteria in mice with IBI

**Table 9. The Effect of Various Treatment on Intestinal Anaerobic Bacteria in Mice with IBI (unit: logCFU/g)**

| Sample # | Normal control group (a) | IBI control group (b) | GLSs high dose treatment group (c) | GLSs medium dose treatment group (d) | GLSs low dose treatment group (e) | *LB* high dose treatment group (f) | *LB* medium dose treatment goup (g) | *LB* low dose treatment group (h) | GLSs-LB high dose treatment group (i) | GLSs-LB medium dose treatment group (j) | GLSs-LB low dose treatment group (k) |
|---|---|---|---|---|---|---|---|---|---|---|---|
| 1 | 11.28 | 8.55 | 11.39 | 11.08 | 10.45 | 11.54 | 10.45 | 10.3 | 11.36 | 10.65 | 9.68 |
| 2 | 11.07 | 8.63 | 11.19 | 11.12 | 10.85 | 11.96 | 11.56 | 10.84 | 12.68 | 11.47 | 10.56 |
| 3 | 11.47 | 8.25 | 11.37 | 12. 41 | 10.63 | 11.53 | 11.85 | 10.36 | 12.63 | 11.01 | 10.12 |
| 4 | 11.24 | 8.54 | 11.22 | 11.56 | 11.04 | 11.85 | 10.58 | 10.42 | 12.49 | 12.31 | 10.34 |
| 5 | 11.77 | 8.43 | 11.12 | 12.10 | 10.56 | 12.03 | 10.56 | 10.34 | 12.64 | 11.62 | 9.98 |
| 6 | 11.56 | 7.96 | 11.37 | 11.49 | 10.96 | 11.55 | 10.46 | 10.23 | 12.51 | 11.42 | 11.25 |
| 7 | 12.96 | 8.25 | 11.49 | 11.23 | 11.23 | 11.85 | 10.74 | 11.38 | 12.32 | 11.31 | 10.45 |
| 8 | 12.12 | 8.12 | 12.04 | 11. 79 | 10.59 | 11.24 | 11.32 | 11.49 | 12.04 | 12.17 | 10.42 |
| 9 | 13.1 | 8.32 | 12.23 | 12.14 | 10.47 | 11.28 | 11.42 | 11.47 | 13.11 | 11.69 | 10.24 |
| 10 | 12.2 | 8.54 | 11.08 | 11.25 | 10.49 | 11.11 | 10.71 | 11.21 | 12.14 | 11.89 | 10.84 |
| Total | 116.77 | 83.59. | 114.9 | 116.17 | 107.27 | 115.94 | 109.06 | 108.04 | 123.92 | 115.54 | 103.88 |
| Average | 11.68 | 8.36 | 11.49 | 11. 62 | 10.73 | 11,59 | 10.91 | 10.80 | 12.39 | 11.56 | 10.39 |

**Table 10. Comparison of the Effect of Various Treatment on Intestinal Anaerobic Bacteria in Mice with IBI (t-test).**

| Groups compared | | Groups compared | | Groups compared | |
|---|---|---|---|---|---|
| a and b | ## | c and d | | e and j | # |
| a and c | | c and e | # | e and k | |
| a and d | | c and f | | f and g | # |
| a and e | # | c and g | # | f and h | # |
| a and f | | c and h | # | f and i | # |
| a and g | # | c and i | # | f and j | |
| a and h | # | c and j | | f and k | # |
| a and i | # | c and k | # | g and h | |
| a and j | | d and e | # | G and i | ## |
| a and k | # | d and f | | g and j | # |
| b and c | # | d and g | | and k | |
| b and d | # | d and h | | h and i | ## |
| b and e | # | d and i | ## | h and j | # |
| b and f | # | d and j | | h and k | |
| b and g | # | d and k | | i and j | # |
| b and h | # | e and f | # | i and k | ## |
| b and i | # | e and g | | j and k | # |
| b and j | # | e and h | | | |
| b and k | # | e and i | ## | | |

| | | | | | |
|---|---|---|---|---|---|
| Note: # is p < 0.05, ## is p < 0.01. | | | | | |

Tables 11 and 12 show the effects of various treatments on intestinal anaerobic bacteria in the IBI prevention model.

**Table 11. The Effects of the Medications Preventing Intestinal Anaerobic Bacteria on Bacteria Imbalances in the Mouse model (unit: logCFU/g)**

| Sample # | Normal control group (a) | IBI control group (b) | GLSs high dose pretreatment group (c) | GLSs medium dose pretreatment group (d) | GLSs low dose pretreatment group (e) | LB high dose pretreatment group (f) | LB medium dose pretreatment group (g) | LB low dose pretreatment group (h) | GLSs-LB high dose pretreatment group (i) | GLSs-LB medium dose pretreatment group (i) | GLSs-LB low dose pretreatment group (k) |
|---|---|---|---|---|---|---|---|---|---|---|---|
| 1 | 11.28 | 8.55 | 11.02 | 10.82 | 10.57 | 10.24 | 10.89 | 9.66 | 11.74 | 10.85 | 10.66 |
| 2 | 11.07 | 8.63 | 10.95 | 10.84 | 10.68 | 10.54 | 10.89 | 9.66 | 11.72 | 10.89 | 10.81 |
| 3 | 11.47 | 8.25 | 10.92 | 10.85 | 10.64 | 10.54 | 10.82 | 9.54 | 11.3 | 10.86 | 10.68 |
| 4 | 11.24 | 8.54 | 11.37 | 10.56 | 10.55 | 10.76 | 10.95 | 9.94 | 11.36 | 10.79 | 11.03 |
| 5 | 11.77 | 8.43 | 11.11 | 10.91 | 10.78 | 10.48 | 10.96 | 10.05 | 11.22 | 10.82 | 10.97 |
| Total | 56.83 | 42.4 | 55.37 | 53.98 | 53.22 | 52.56 | 54.51 | 48.85 | 57.34 | 54.21 | 54.15 |
| Average | 11.366 | 8.48 | 11.074 | 10.796 | 10.644 | 10.512 | 10.902 | 9.77 | 11.468 | 10.842 | 10.83 |

**Table 12. Comparison of the Effects of Various Treatments on Intestinal Anaerobic Bacteria in IBI Prevention Model (t-test)**

| Groups compared | | Groups compared | |
|---|---|---|---|
| a and b | ## | d and f | |
| a and c | | d and g | |
| a and d | ## | d and h | ## |
| a and e | ## | d and i | ## |
| a and f | ## | d and j | # |
| a and g | # | d and k | |
| a and h | ## | e and f | |
| a and i | | e and g | ## |
| a and j | # | e and h | ## |
| a and k | # | e and i | ## |
| b and c | ## | e and j | ## |
| b and d | ## | e and k | |
| b and e | ## | f and g | ## |
| b and f | ## | f and h | ## |
| b and g | ## | f and i | ## |
| b and h | ## | f and j | # |
| b and i | ## | f and k | ## |
| b and j | ## | g and h | ## |
| b and k | ## | g and i | ## |
| c and d | | g and j | |
| c and e | # | g and k | |
| c and f | ## | h and i | ## |
| c and g | | h and j | ## |
| c and h | ## | h and k | ## |
| c and i | | i and j | ## |
| c and j | | i and k | # |
| c and k | ## | j and k | |
| d and e | # | | |

| | | | |
|---|---|---|---|
| Note: # is p < 0.05, ## is p < 0.01. | | | |

*Changes in intestinal aerobic bacteria:* Tables 13 and 14 show the effects of the various treatments on intestinal aerobic bacteria: in normal Mice.

**Table 13. The Effects of Various Treatment on Intestinal Aerobic Bacteria in Normal Mice (unit: logCFU/g).**

| Sample # | Normal control group (a) | IBI control goup (b) | GLSs high dose control group (c) | GLSs medium dose control goup (d) | GLSs low dose control goup (e) | *LB* high dose control group (f) | *LB* medium dose control goup (g) | *LB* low dose control group (h) | GLSs-LB high dose control group (i) | GLSs-LB medium dose control group (j) | GLSs-LB low dose control group (k) |
|---|---|---|---|---|---|---|---|---|---|---|---|
| 1 | 9.67 | 6.27 | 11.47 | 10.32 | 8.69 | 11.07 | 10.2 | 8.23 | 11.88 | 10.92 | 9.13 |
| 2 | 9.27 | 6.45 | 11.56 | 10.34 | 8.34 | 11.34 | 10.56 | 8.14 | 11.59 | 11.56 | 8.84 |
| 3 | 9.61 | 6.89 | 11.36 | 11.23 | 9.13 | 11.12 | 11.02 | 9.09 | 11.74 | 11.56 | 9.22 |
| 4 | 9.73 | 6.57 | 11.74 | 11.56 | 9.31 | 11.13 | 11.12 | 9.21 | 11.96 | 11.89 | 9.29 |
| 5 | 9.8 | 7.28 | 10.89 | 10.31 | 8.33 | 10.62 | 10.61 | 8.44 | 11.98 | 11.52 | 8.84 |
| 6 | 9.61 | 6.31 | 11.35 | 10.34 | 8.22 | 11.31 | 10.11 | 8.08 | 11.56 | 10.49 | 8.63 |
| 7 | 9.23 | 6.11 | 11.47 | 10.11 | 8.35 | 11.03 | 10.02 | 8.17 | 11.87 | 10.98 | 8.34 |
| 8 | 9.14 | 6.23 | 10.69 | 11.43 | 8.49 | 10.48 | 11.14 | 8.15 | 11.95 | 11.53 | 8.63 |
| 9 | 9.18 | 6.25 | 10.74 | 10.32 | 8.47 | 10.36 | 10.17 | 8.25 | 11.54 | 10.84 | 8.94 |
| 10 | 9.47 | 7.21 | 11.42 | 10.45 | 8.38 | 11.01 | 9.38 | 8.31 | 11.89 | 10.42 | 8.65 |
| Total | 94.71 | 65.57 | 112.69 | 106.41 | 85.71 | 118.84 | 109.06 | 83.80 | 117.96 | 111.71 | 88.51 |
| Average | 9.47 | 6.56 | 11.27 | 10.64 | 8.57 | 11.88 | 10.91 | 8.38 | 11.80 | 11.17 | 8.85 |

**Table 14. Comparison of the Effects of the Various Medications on Intestinal Aerobic Bacteria (t-test).**

| Groups compared | | Groups compared | | Groups compared | |
|---|---|---|---|---|---|
| a and b | ## | c and d | # | e and j | # |
| a and c | ## | c and e | # | e and k | # |
| a and d | # | c and f | # | f and g | |
| a and e | | c and g | # | f and h | # |
| a and f | ## | c and h | ## | f and i | |
| a and g | # | c and I | | f and j | |
| a and h | # | c and j | | f and k | # |
| a and i | ## | c and k | ## | g and h | # |
| a and j | ## | d and e | # | g and i | |
| a and k | # | d and f | # | g and j | |
| b and c | ## | d and g | # | g and k | # |
| b and d | ## | d and h | # | h and i | ## |
| b and e | ## | d and I | # | h and j | ## |
| b and f | ## | d and j | # | h and k | |
| b and g | ## | d and k | # | i and j | |
| b and h | ## | e and f | # | i and k | ## |
| b and i | ## | e and g | # | j and k | ## |
| b and j | ## | e and h | # | | |
| b and k | ## | e and i | # | | |

| | | | | | |
|---|---|---|---|---|---|
| Note:#isp<0.05, ## is p<0.01. | | | | | |

Tables 15 and 16 show the effects of various treatments on intestinal aerobic bacteria in mice with IBI:

**Table 15. The Effects of Various Treatment on Intestinal Aerobic Bacteria in Mice with IBI (unit: logCFU/g)**

| Sample # | Normal control group (1) | IBI control group (2) | GLSs high dose treatment group (3) | GLSs medium dose treatment group (4) | GLSs low dose treatment group (5) | *LB* high dose treatment group (6) | *LB* medium dose treatment group (7) | *LB* low dose treatment group (8) | GLSs-LB high dose treatment group (9) | GLSs-LB medium dose treatmen group (10) | GLSs-LB low dose treatme t group (11) |
|---|---|---|---|---|---|---|---|---|---|---|---|
| A | 9.067 | 6.27 | 9.61 | 8.28 | 8.61 | 8.81 | 8.55 | 8.81 | 9.53 | 9.34 | 9.65 |
| B | 9.27 | 6.45 | 9.64 | 8.65 | 8.73 | 8.85 | 8.56 | 8.84 | 9.61 | 9.4 | 9.71 |
| C | 9.61 | 6.89 | 9.76 | 8.78 | 8.83 | 8.88 | 8.83 | 9.13 | 9.62 | 9.398 | 9.87 |
| D | 9.73 | 6.57 | 9.94 | 9.45 | 8.88 | 9.11 | 8.86 | 9.28 | 9.74 | 9.46 | 9.897 |
| E | 9.8 | 7.28 | 10.06 | 9.43 | 9.01 | 9.18 | 8.69 | 9.44 | 9.77 | 9.88 | 10.37 |
| Average value | 9.5 | 6.7 | 9.81 | 8.92 | 8.82 | 8.97 | 8.7 | 9.1 | 9.66 | 9.5 | 9.9 |

**Table 16. Comparison of the Effects of Various Treatments on Intestinal Aerobic Bacteria in Mice with IBI (t-test)**

| Groups compared | | Groups compared | |
|---|---|---|---|
| 1 and 11 | ## | 3 and 6 | ## |
| 1 and 10 | | 3 and 5 | ## |
| 1 and 9 | | 3 and 4 | ## |
| 1 and 8 | ## | 4 and 11 | ## |
| 1 and 7 | ## | 4 and 10 | # |
| 1 and 6 | ## | 4 and 9 | ## |
| 1 and 5 | ## | 4 and 8 | |
| 1 and 4 | ## | 4 and 7 | |
| 1 and 3 | # | 4 and 6 | |
| 1 and 2 | ## | 4 and 5 | |
| 2 and 11 | ## | 5 and 11 | ## |
| 2 and 10 | ## | 5 and 10 | ## |
| 2 and 9 | ## | 5 and 9 | ## |
| 2 and 8 | ## | 5 and 8 | ## |
| 2 and 7 | ## | 5 and 7 | |
| 2 and 6 | ## | 5 and 6 | ## |
| 2 and 5 | ## | 6 and 11 | ## |
| 2 and 4 | ## | 6 and 10 | ## |
| 2 and 3 | ## | 6 and 9 | ## |
| 3 and 11 | | 6 and 8 | |
| 3 and 10 | ## | 6 and 7 | # |
| 3 and 9 | # | 7 and 11 | ## |
| 3 and 8 | ## | 7 and 10 | ## |
| 3 and 7 | ## | | |

| | | | |
|---|---|---|---|
| Note: # is p < 0.05, ## is p < 0.01 | | | |

Tables 17 and 18 show the effects of various treatments on intestinal aerobic bacteria in the IBI prevention model.

**Table 17. The Effects of Various Treatments on Intestinal Aerobic Bacteria in the IBI Prevention Model (unit: logCFU/g)**

| Sample # | Normal control group (1) | IBI control group (2) | GLSs high dose pretreatment group (3) | GLSs medium dose pretreatment group (4) | GLSs low dose pretreatment group (5) | *LB* high dose pretreatment group (6) | *LB* medium dose pretreatment group (7) | *LB* low dose pretreatment group (8) | GLSs-LB high dose pretreatment group (9) | GLSs-LB medium dose pretreatment group (10) | GLSs-LB low dose pretreatment group (11) |
|---|---|---|---|---|---|---|---|---|---|---|---|
| A | 9.067 | 6.27 | 7.94 | 8.86 | 9.26 | 9.25 | 9.70 | 8.64 | 8.73 | 8.68 | 8.43 |
| B | 9.27 | 6.45 | 7.82 | 9.26 | 9.22 | 9.29 | 9.66 | 8.68 | 8.75 | 8.68 | 8.46 |
| C | 9.61 | 6.89 | 7.74 | 9.3 | 9.24 | 9.29 | 9.84 | 8.74 | 8.988 | 8.71 | 8.589 |
| D | 9.73 | 6.57 | 7.69 | 9.6 | 9.41 | 9.38 | 9.88 | 8.76 | 9.22 | 8.81 | 8.589 |
| E | 9.8 | 7.28 | 7.85 | 8.47 | 9.44 | 9.48 | 9.94 | 8.83 | 9.39 | 8.66 | 8.67 |
| Average value | 9.5 | 6.7 | 7.81 | 9.1 | 9.32 | 9.34 | 9.81 | 8.53 | 9.02 | 8.71 | 8.55 |

**Table 18. Comparison of the Effects of Various Treatments on Intestinal Aerobic Bacteria in the IBI Prevention Model (t-test)**

| Groups compared | | Groups compared | |
|---|---|---|---|
| 1 and 11 | ## | 4 and 10 | |
| 1 and 10 | ## | 4 and 9 | |
| 1 and 9 | ## | 4 and 8 | |
| 1 and 8 | ## | 4 and 7 | # |
| 1 and 7 | # | 4 and 6 | |
| 1 and 6 | | 4 and 5 | |
| 1 and 5 | | 5 and 11 | ## |
| 1 and 4 | | 5 and 10 | ## |
| 1 and 3 | ## | 5 and 9 | # |
| 1 and 2 | ## | 5 and 8 | ## |
| 2 and 11 | ## | 5 and 7 | ## |
| 2 and 10 | ## | 5 and 6 | |
| 2 and 9 | ## | 6 and 11 | ## |
| 2 and 8 | ## | 6 and 10 | ## |
| 2 and 7 | ## | 6 and 9 | # |
| 2 and 6 | ## | 6 and 8 | |
| 2 and 5 | ## | 6 and 7 | ## |
| 2 and 4 | ## | 7 and 11 | ## |
| 2 and 3 | ## | 7 and 10 | ## |
| 3 and 11 | | 7 and 9 | ## |
| 3 and 10 | ## | 7 and 8 | ## |
| 3 and 9 | ## | 8 and 11 | # |
| 3 and 8 | ## | 8 and 10 | |
| 3 and 7 | ## | 8 and 9 | # |
| 3 and 6 | ## | 9 and 11 | |
| 3 and 5 | ## | 9 and 10 | |
| 3 and 4 | ## | 10 and 11 | # |
| 4 and 11 | | | |

| | | | |
|---|---|---|---|
| Note: # is p < 0.05, ## is p 0.01 | | | |

*Pathological changes:* Intestine samples from mice of various groups were collected, the stool in the intestine was extracted. The intestine samples were washed with aseptic normal saline, fixed in normal formalin, embedded in paraffin and sectioned. HE staining was performed and pathological changes were observed under a light microscope. Intestinal mucosa congestion was evident in mice from the IBI group but not evident in mice from normal control group and GLSs-BL treatment group.

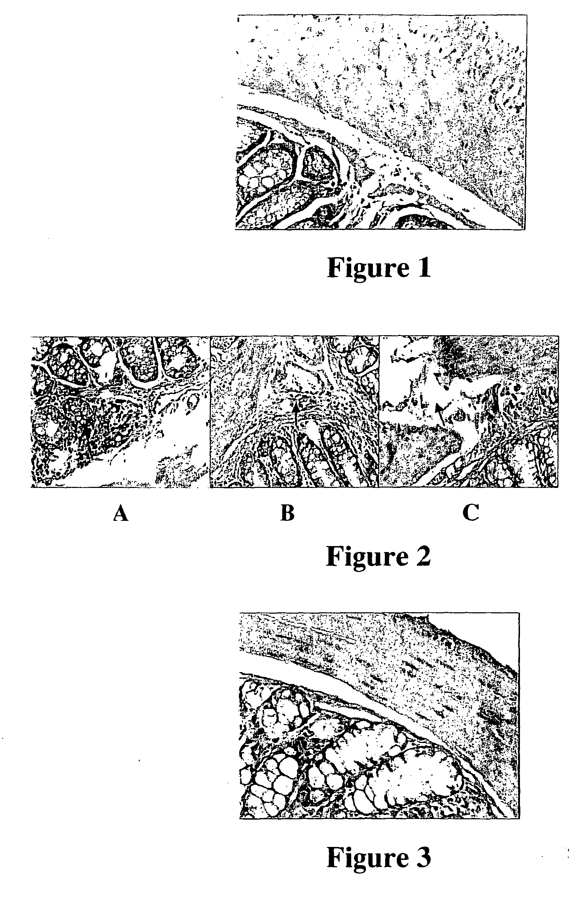

Figure 1 shows a representative intestinal mucosa section from a mouse in the normal control group. In the mucosal layer, the tissue structure is normal. No inflammatory cell infiltration, erosion, necrotic shedding, vasodilation, edema, bleeding, ulceration or other pathological changes were observed. In the submucosal layer, the tissue structure is normal with no edema, vasodilation, inflammatory cell infiltration or other pathological changes. In the muscle and serous membrane layer, the tissue structure is also normal with no inflammatory cell infiltration, degenerative necrosis or other pathological changes.
Figure 2 shows representative intestinal mucosa sections from a mouse in the IBI control group. Panel A shows inflammatory cell infiltration and erosion in the mucosal layer. Panel B shows vasodilation and inflammatory cell infiltration in the submucosal layer. Panel C shows degeneration and inflammatory cell infiltration in the muscle layer.
Figure 3 shows a representative intestinal mucosa section from a mouse in the GLSs low dose control group. In the mucosal layer, the tissue structure is normal with no inflammatory cell infiltration, erosion, necrotic shedding, vasodilation, edema, bleeding, ulceration or other pathological changes. In the submucosal layer, the tissue structure is normal with no edema, vasodilation, inflammatory cell infiltration or other pathological changes. In the muscle layer, the tissue structure is normal with no inflammatory cell infiltration, degenerative necrosis or other pathological changes. In the serous membrane layer, the tissue structure is normal with no inflammatory cell infiltration, degenerative necrosis or other pathological changes.

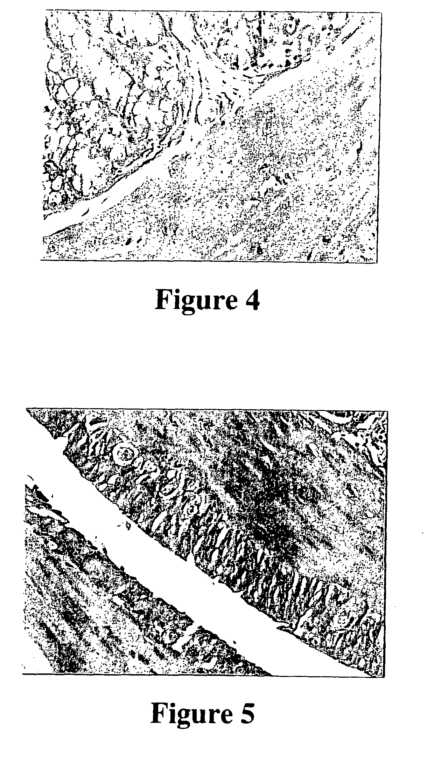

Figure 4 shows a representative intestinal mucosa section from a mouse in the GLSs medium dose control group. In the mucosal layer, the tissue structure is normal with no inflammatory cell infiltration, erosion, necrotic shedding, vasodilation, edema, bleeding, ulceration or other pathological changes. In the submucosal layer, the tissue structure is normal with no edema, vasodilation, inflammatory cell infiltration or other pathological changes. In the muscle layer, the tissue structure is normal, no inflammatory cell infiltration, degenerative necrosis or other pathological changes. In the serous membrane layer, the tissue structure is normal, no inflammatory cell infiltration, degenerative necrosis or other pathological changes.
Figure 5 shows a representative intestinal mucosa section from a mouse in the GLSs high dose control group. In the mucosal layer, the tissue structure is normal with no inflammatory cell infiltration, erosion, necrotic shedding, vasodilation, edema, bleeding, ulceration or other pathological changes. In the submucosal layer, the tissue structure is normal with no edema, vasodilation, inflammatory cell infiltration or other pathological changes. In the muscle layer, the tissue structure is normal, no inflammatory cell infiltration, degenerative necrosis or other pathological changes. In the serous membrane layer, the tissue structure is normal, no inflammatory cell infiltration, degenerative necrosis or other pathological changes.

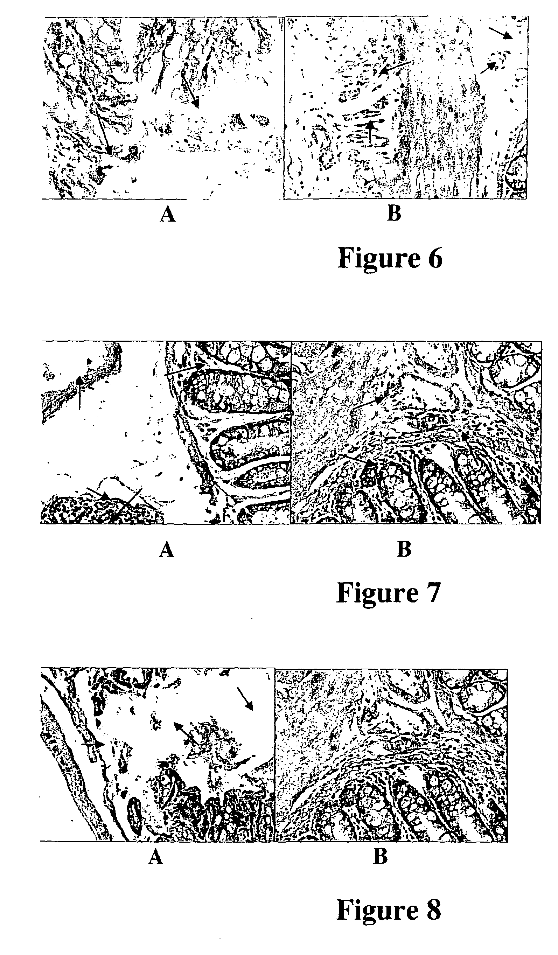

Figure 6 shows representative intestinal mucosa sections from a mouse in the LB low dose control group. Panel A shows a slight degree of inflammatory cell infiltration and erosion in the mucosal layer. Panel B shows a slight degree of vasodilation and inflammatory cell infiltration in the submucosal layer, as well as a slight degree of degeneration and inflammatory cell infiltration at the muscle layer.
Figure 7 shows representative intestinal mucosa sections from a mouse in the LB medium dose control group. Panel A shows inflammatory cell infiltration and erosion in the mucosal layer, as well as vasodilation and inflammatory cell infiltration in the submucosal layer. Panel B shows a slight degree of vasodilation and inflammatory cell infiltration at the submucosal layer, as well as a slight degree of degeneration and inflammatory cell infiltration in the muscle layer.
Figure 8 shows representative intestinal mucosa sections from a mouse in the LB high dose control group. Panel A shows inflammatory cell infiltration and erosion in the mucosal layer. Panel B shows vasodilation and inflammatory cell infiltration in the submucosal layer, as well as degeneration and inflammatory cell infiltration in the muscle layer.

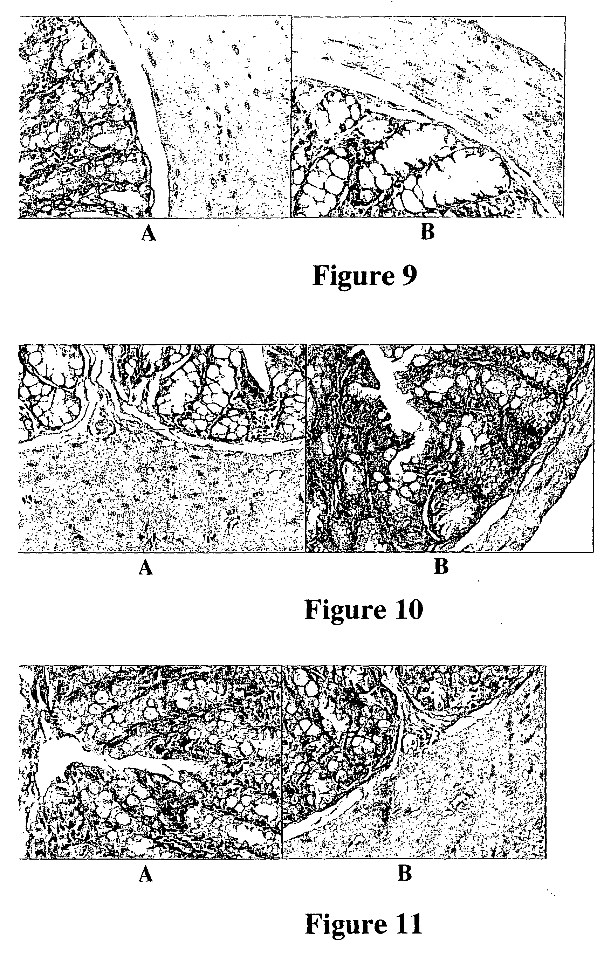

Figure 9 shows representative intestinal mucosa sections from a mouse in the GLSs -LB low dose control group. In the mucosal layer, the tissue structure is normal with no inflammatory cell infiltration, erosion, necrotic shedding, vasodilation, edema, bleeding, ulceration or other pathological changes. In the submucosal layer, the tissue structure is normal with no edema, vasodilation, inflammatory cell infiltration or other pathological changes. In the muscle layer, the issue structure is normal with no inflammatory cell infiltration, degenerative necrosis or other pathological changes. In the serous membrane layer, the tissue structure is normal with no inflammatory cell infiltration, degenerative necrosis or other pathological changes.
Figure 10 shows representative intestinal mucosa sections from a mouse in the GLSs -LB medium dose control group. In the mucosal layer, the tissue structure is normal with no inflammatory cell infiltration, erosion, necrotic shedding, vasodilation, edema, bleeding, ulceration or other pathological changes. In the submucosal layer, the tissue structure is normal with no edema, vasodilation, inflammatory cell infiltration or other pathological changes. In the muscle layer, the tissue structure is normal with no inflammatory cell infiltration, degenerative necrosis or other pathological changes.
Figure 11 shows representative intestinal mucosa sections from a mouse in the GS-LB high dose control group. In the mucosal layer, the tissue structure is normal with no inflammatory cell infiltration, erosion, necrotic shedding, vasodilation, edema, bleeding, ulceration or other pathological changes. In the submucosal layer, the tissue structure is normal, no edema, vasodilation, inflammatory cell infiltration or other pathological changes. In the muscle layer, the tissue structure is normal with no inflammatory cell infiltration, degenerative necrosis or other pathological changes.

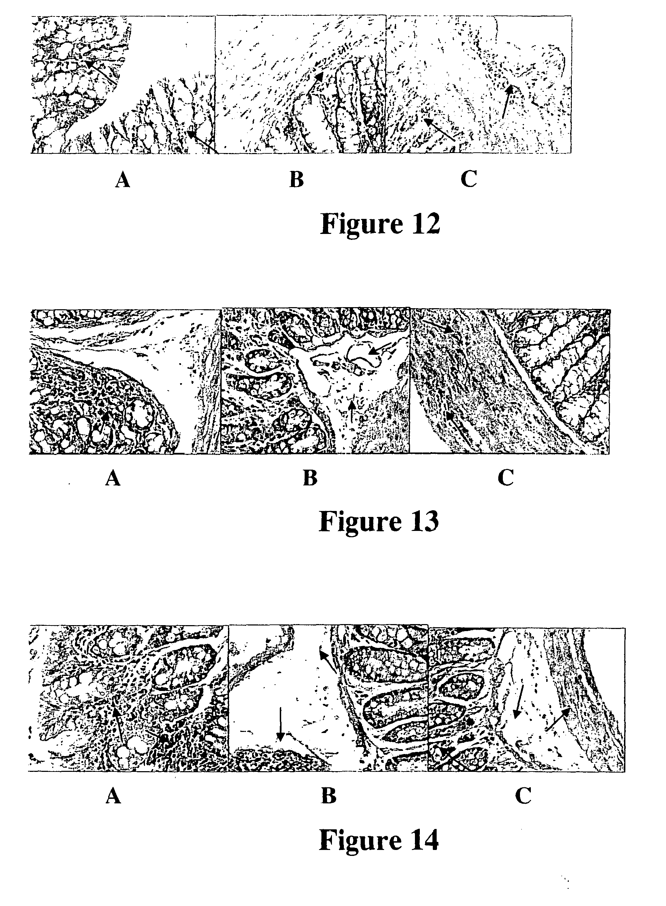

Figure 12 shows representative intestinal mucosa sections from a mouse in the LB low dose treatment group. Panel A shows inflammatory cell infiltration at the mucosal layer. Panel B shows vasodilation and inflammatory cell infiltration at the submucosal layer. Panel C shows inflammatory cell infiltration at the muscle layer.
Figure 13 shows representative intestinal mucosa sections from a mouse in the LB medium dose treatment group. Panel A shows inflammatory cell infiltration at the mucosal layer. Panel B shows vasodilation and inflammatory cell infiltration at the submucosal layer. Panel C shows inflammatory cell infiltration at the muscle layer.
Figure 14 shows representative intestinal mucosa sections from a mouse in the LB high dose treatment group. Panel A shows inflammatory cell infiltration at the mucosal layer. Panel B shows vasodilation, inflammatory cell infiltration and edema at the submucosal layer. Panel C shows inflammatory cell infiltration at the muscle layer.

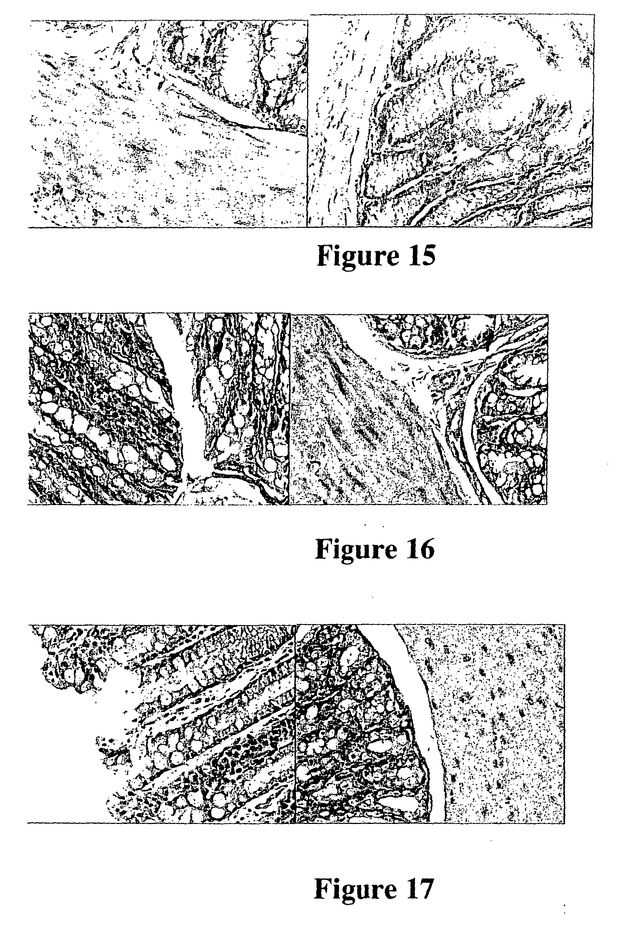

Figure 15 shows representative intestinal mucosa sections from a mouse in the GLSs low dose treatment group. In the mucosal layer, tissue structure is normal with no inflammatory cell infiltration, erosion, necrotic shedding, vasodilation, edema, bleeding, ulceration or other pathological changes. In the submucosal layer, there is no inflammatory cell infiltration, edema or other pathological changes. In the muscle layer, there is no inflammatory cell infiltration, degenerative necrosis or other pathological changes. In the serous membrane layer, the tissue structure is normal with no inflammatory cell infiltration, degenerative necrosis or other pathological changes.
Figure 16 shows representative intestinal mucosa sections from a mouse in the GLSs medium dose treatment group. In the mucosal layer, the tissue structure is normal with no inflammatory cell infiltration, erosion, necrotic shedding, vasodilation, edema, bleeding, ulceration or other pathological changes. In the submucosal layer, there is no inflammatory cell infiltration, no edema or other pathological changes. In the muscle layer, there is no inflammatory cell infiltration, no degenerative necrosis or other pathological changes. In the serous membrane layer, the tissue structure is normal with no inflammatory cell infiltration, degenerative necrosis or other pathological changes.
Figure 17 shows representative intestinal mucosa sections from a mouse in the GLSs high dose treatment group. In the mucosal layer, the tissue structure is normal with no inflammatory cell infiltration, erosion, necrotic shedding, vasodilation, edema, bleeding, ulceration or other pathological changes. In the submucosal layer, there is no inflammatory cell infiltration, no edema or other pathological changes. In the muscle layer, there is no inflammatory cell infiltration, no degenerative necrosis or other pathological changes. In the serous membrane layer, the tissue structure is normal with no inflammatory cell infiltration, degenerative necrosis or other pathological changes.

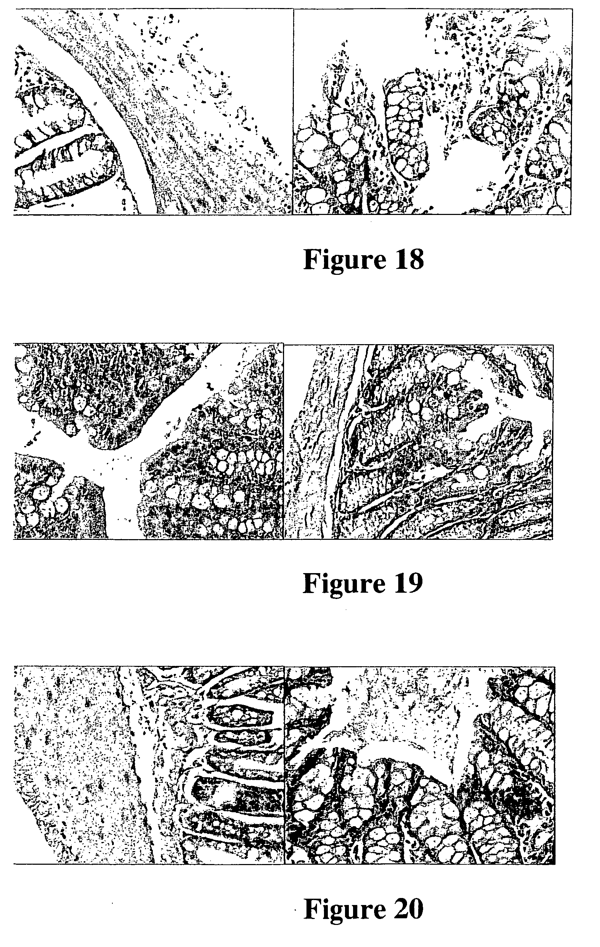

Figure 18 shows representative intestinal mucosa sections from a mouse in the GLSs-LB low dose treatment group. In the mucosal layer, the tissue structure is normal with no inflammatory cell infiltration, erosion, necrotic shedding, vasodilation, edema, bleeding, ulceration or other pathological changes. In the submucosal layer, there is no inflammatory cell infiltration, no edema or other pathological changes. In the muscle layer, there is no inflammatory cell infiltration, no degenerative necrosis or other pathological changes. In the serous membrane layer, the tissue structure is normal with no inflammatory cell infiltration, degenerative necrosis or other pathological changes.
Figure 19 shows representative intestinal mucosa sections from a mouse in the GLSs-LB medium dose treatment group. In the mucosal layer, the tissue structure is normal with no inflammatory cell infiltration, erosion, necrotic shedding, vasodilation, edema, bleeding, ulceration or other pathological changes. In the submucosal layer, there is no inflammatory cell infiltration, no edema or other pathological changes. In the muscle layer, there is no inflammatory cell infiltration, no degenerative necrosis or other pathological changes. In the serous membrane layer, the tissue structure is normal with no inflammatory cell infiltration, degenerative necrosis or other pathological changes.
Figure 20 shows representative intestinal mucosa sections from a mouse in the GLSs-LB high dose treatment group. In the mucosal layer, the tissue structure is normal with no inflammatory cell infiltration, erosion, necrotic shedding, vasodilation, edema, bleeding, ulceration or other pathological changes. In the submucosal layer, there is no inflammatory cell infiltration, no edema or other pathological changes. In the muscle layer, there is no inflammatory cell infiltration, no degenerative necrosis or other pathological changes. In the serous membrane layer, the tissue structure is normal with no inflammatory cell infiltration, degenerative necrosis or other pathological changes.

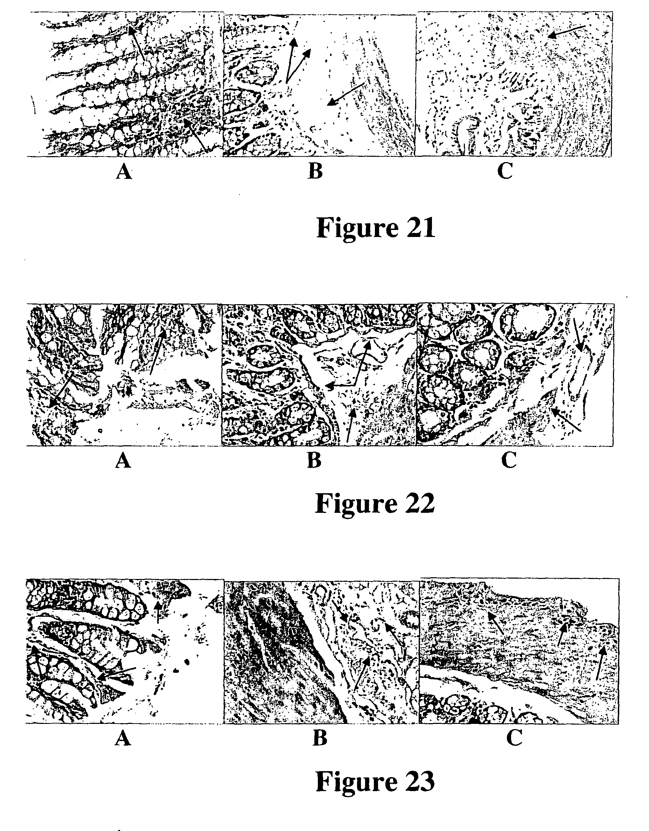

Figure 21 shows representative intestinal mucosa sections from a mouse in the LB low dose prevention group. Panel A shows inflammatory cell infiltration in the mucosal layer. Panel B shows vasodilation and inflammatory cell infiltration in the submucosal layer. Panel C shows inflammatory cell infiltration in the muscle layer.
Figure 22 shows representative intestinal mucosa sections from a mouse in the LB medium dose prevention group. Panel A shows inflammatory cell infiltration and erosion in the mucosal layer. Panel B shows vasodilation, inflammatory cell infiltration and edema in the submucosal layer. Panel C shows inflammatory cell infiltration in the muscle layer.
Figure 23 shows representative intestinal mucosa sections from a mouse in the LB high dose prevention group. Panel A shows inflammatory cell infiltration and erosion in the mucosal layer. Panel B shows vasodilation, inflammatory cell infiltration and edema in the submucosal layer. Panel C shows inflammatory cell infiltration in the muscle layer.

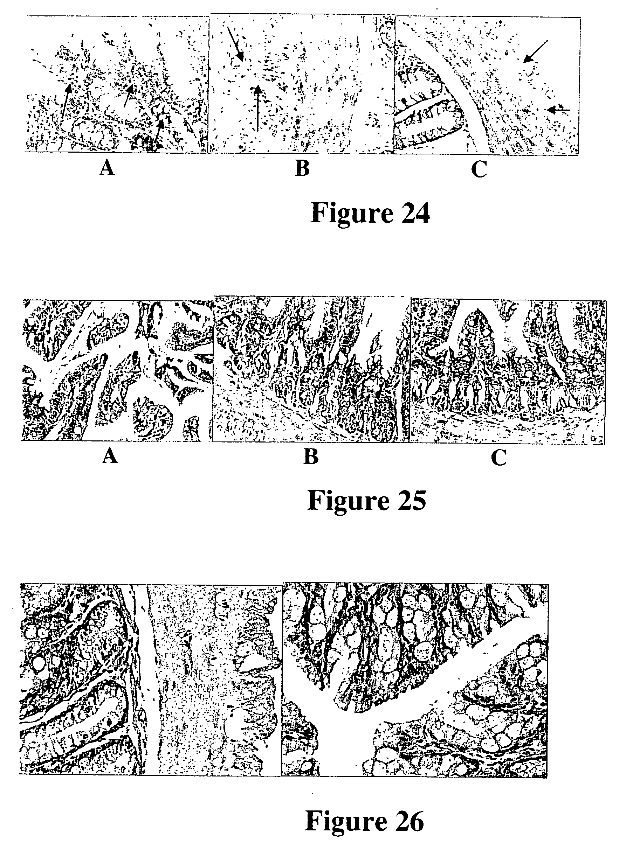

Figure 24 shows representative intestinal mucosa sections from a mouse in the GLSs low dose prevention group. Panel A shows slight degree of inflammatory cell infiltration in the mucosal layer, but there is no erosion, necrotic shedding, vasodilation, edema, bleeding, ulceration or other pathological changes. Panel B shows slight degree of inflammatory cell infiltration in the submucosal layer. Panel C shows slight degree of inflammatory cell infiltration in the muscle layer, but there is no degenerative necrosis or other pathological changes.
Figure 25 shows representative intestinal mucosa sections from a mouse in the GLSs medium dose prevention group. Panel A shows that in the mucosal layer, tissue structure is normal with no inflammatory cell infiltration, erosion, necrotic shedding, vasodilation, edema, bleeding, ulceration or other pathological changes. Panel B shows that there is no inflammatory cell infiltration, no edema or other pathological changes in the submucosal layer. Panel C shows that there is no inflammatory cell infiltration, no degenerative necrosis or other pathological changes in the muscle layer.
Figure 26 shows representative intestinal mucosa sections from a mouse in the GLSs high dose prevention group. In the mucosal layer, the tissue structure is normal with no inflammatory cell infiltration, erosion, necrotic shedding, vasodilation, edema, bleeding, ulceration or other pathological changes. In the submucosal layer, there is no inflammatory cell infiltration, no edema or other pathological changes. In the muscle layer, there is no inflammatory cell infiltration, no degenerative necrosis or other pathological changes. In the serous membrane layer, the tissue structure is normal with no inflammatory cell infiltration, degenerative necrosis or other pathological changes.

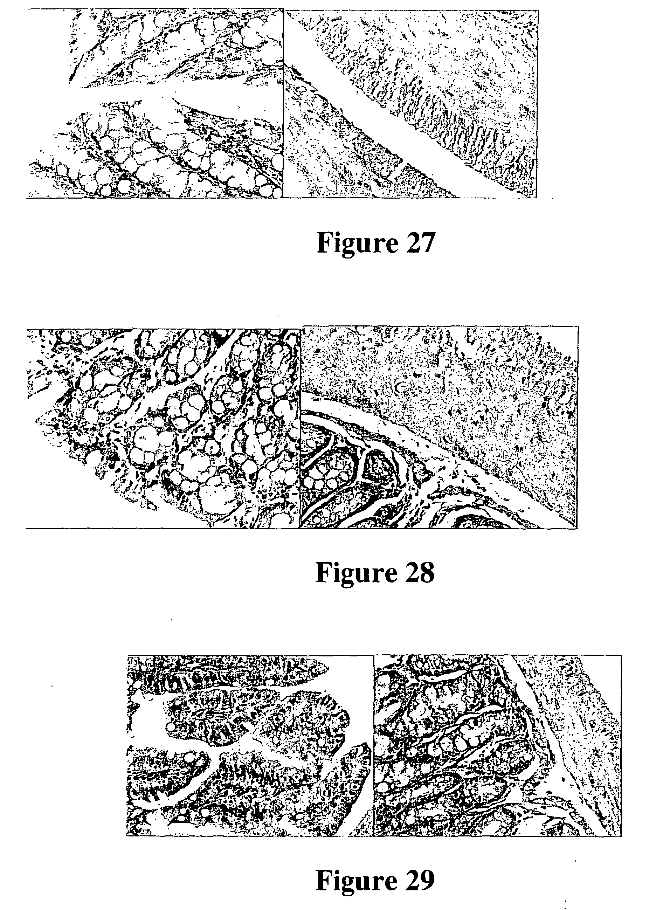

Figure 27 shows representative intestinal mucosa sections from a mouse in the GLSs-LB low dose prevention group. In the mucosal layer, the tissue structure is normal with no inflammatory cell infiltration, erosion, necrotic shedding, vasodilation, edema, bleeding, ulceration or other pathological changes. In the submucosal layer, there is no inflammatory cell infiltration, no edema or other pathological changes. In the muscle layer, there is no inflammatory cell infiltration, no degenerative necrosis or other pathological changes. In the serous membrane layer, the tissue structure is normal with no inflammatory cell infiltration, degenerative necrosis or other pathological changes.
Figure 28 shows representative intestinal mucosa sections from a mouse in the GLSs-LB medium dose prevention group. In the mucosal layer, the tissue structure is normal with no inflammatory cell infiltration, erosion, necrotic shedding, vasodilation, edema, bleeding, ulceration or other pathological changes. In the submucosal layer, there is no inflammatory cell infiltration, no edema or other pathological changes. In the muscle layer, there is no inflammatory cell infiltration, no degenerative necrosis or other pathological changes. In the serous membrane layer, the tissue structure is normal, no inflammatory cell infiltration, degenerative necrosis or other pathological changes.
Figure 29 shows representative intestinal mucosa sections from a mouse in the GLSs-LB high dose prevention group. In the mucosal layer, the tissue structure is normal with no inflammatory cell infiltration, erosion, necrotic shedding, vasodilation, edema, bleeding, ulceration or other pathological changes. In the submucosal layer, there is no inflammatory cell infiltration, no edema or other pathological changes. In the muscle layer, there is no inflammatory cell infiltration, no degenerative necrosis or other pathological changes. In the serous membrane layer, the tissue structure is normal with no inflammatory cell infiltration, degenerative necrosis or other pathological changes.

### III. Conclusion

The mice in the normal control group showed gains in body weight. The mice in the IBI control group did not show gains in body weight. The mice subjected to *Lactobacillus bifidus* treatment alone also did not show gains in body weight. The mice subjected to GLSs treatment alone and the mice subjected GLSs*-Lactobacillus bifidus* mixture treatment showed visible gains in body weight. The increase of bodyweight correlated to the dose of GLSs.

GLSs, *Lactobacillus bifidus* and *GLSs-Lactobacillus bifidus* mixture raise the levels of intestinal anaerobic and aerobic bacteria in the mouse IBI model. The best results were obtained with the *GLSs-Lactobacillus bifidus* mixture. The results of GLSs administration are superior to the results of *Lactobacillus bifidus* administration.

Examination of intestinal samples revealed that mice in the IBI group and the LB treatment/pretreatment groups had congestion in the intestinal mucosa, while mice in the normal control group, the GLSs treatment/pretreatment groups and GLSs-LB treatment/pretreatment groups did not show congestion.

Pathological examination of intestine sections indicated that the intestinal mucosa layers of the mice in the IBI group and the LB treatment/pretreatment groups had inflammatory cell infiltration, erosion and bleeding; the submucosal layer had a slight degree of vasodilation and inflammatory cell infiltration; the muscle layer and serous membrane layer also had pathological changes. In contrast, mice in the normal control group, the GLSs treatment/pretreatment groups, and GLSs-LB treatment/pretreatment groups all showed no pathological changes in the intestinal mucosal layer.

The result show that GLSs, *Lactobacillus bifidus* and GLSs*-Lactobacillus bifidus* mixture may raise aerobic and anaerobic bacteria counts in the intestine of the mouse model, and have a definite elevating effect on the bacterial counts reduced as the result of antibiotics. However, *Lactobacillus bifidus* treatment or pretreatment alone failed to restore the body weight to normal levels, and caused congestion in the intestinal mucosa. Pathological examination also indicates that mice in the IBI control and LB treatment/pretreatment groups presented inflammatory cell infiltration, erosion and bleeding in the intestinal mucosa layer; a slight degree of vasodilation and inflammatory cell infiltration in the submucosal layer showed; and pathological changes in the muscle layer and the serous membrane layer. In contrast, mice in the GLSs treatment/pretreatment and GLSs-LB treatment/pretreatment groups showed significant gain in body weight and no pathological changes in intestine by both macroscopic or microscopic examination. Accordingly, administration of GLSs or *GLSs-Lactobacillus bifidus* mixture provided better results than administration of *Lactobacillus bifidus* in treating or preventing IBI.

The ebodiments illustrated and discussed in this specification are intended only to teach those skilled in the art the best way known to the inventors to make and use the invention. Nothing in this specification should be considered as limiting the scope of the present invention. The above-described embodiments of the invention may be modified or varied, and elements added or omitted, without departing from the invention, as appreciated by those skilled in the art in light of the above teachings. It is therefore to be understood that, within the scope of the claims and their equivalents, the invention may be practiced otherwise than as specifically described.

## Claims

1. Germination activated sporoderm-broken *Ganoderma lucidum* spores (GLSs) for preventing and/or treating an intestinal bacterial imbalance (IBI) in a mammal.

2. The *Ganoderma lucidum* spores (GLSs) according to claim 1, wherein said mammal is a human.

3. The *Ganoderma lucidum* spores (GLSs) according to claim 1, wherein said mammal is a rodent.

4. The *Ganoderma lucidum* spores (GLSs) according to anyone of claims 1 to 3, wherein said IBI is caused by an antibiotic treatment.

5. The *Ganoderma lucidum* spores (GLSs) according to claim 4, wherein said antibiotic is lincomycin hydrochloride.

6. The *Ganoderma lucidum* spores (GLSs) according to anyone of the preceding claims, wherein said GLSs is for administration in an amount of between 0.005 and 20 g / kg body weight / day.

7. The *Ganoderma lucidum* spores (GLSs) according to anyone of the preceding claims, wherein *Ganoderma lucidum* spores (GLSs) is for use in co-administration with beneficial intestinal bacteria.

8. The *Ganoderma lucidum* spores (GLSs) according to claim 7, wherein said beneficial intestinal bacteria are *Lactobacilli* or *Bifidobacteria* or a combination thereof.

9. The *Ganoderma lucidum* spores (GLSs) according to claim 7, wherein said beneficial intestinal bacteria are *Lactobacillus bifidus.*

10. The *Ganoderma lucidum* spores (GLSs) of anyone of claims 7 to 9, wherein the amount of said co-administrated beneficial intestinal bacteria is between 0.02 and 20 g /kg body weight / day.

11. The *Ganoderma lucidum* spores (GLSs) according to claim 1, wherein said GLSs raise aerobic and anaerobic bacterial counts in an intestine of said mammal.

12. The *Ganoderma lucidum* spores (GLSs) according to claim 7, wherein said GLSs and said beneficial intestinal bacteria raise aerobic and anaerobic bacterial counts in an intestine of said mammal.

13. A use of germination activated sporoderm-broken *Ganoderma lucidum* spores (GLSs) for the manufacture of a medicament for preventing and/or treating an intestinal bacterial imbalance (IBI) in a mammal.

14. The use according to claim 13, wherein said mammal has a tendency to develop said IBI, in particular by being.under antibiotic treatment.

15. The use according to claim 14, wherein said GLSs are administered to said mammal prior to or concurrently with said antibiotic treatment.

16. The use according to anyone of claims 13 to 15, wherein said medicament is used in co-administration with beneficial intestinal bacteria.

17. The use according to claim 16, wherein said beneficial intestinal bacteria are *Lactobacilli* or *Bifidobacteria* or a combination thereof.

18. The use according to claim 16, wherein said beneficial intestinal bacteria are *Lactobacillus bifidus.*

19. The use according to claim 13, wherein said GLSs raise aerobic and anaerobic bacterial counts in an intestine of said mammal.

20. The use according to claim 16, wherein said GLSs and said beneficial intestinal bacteria raise aerobic and anaerobic bacterial counts in an intestine of said mammal.
